(19) Europäisches Patentamt
European Patent Office
Office européen des brevets

(11) **EP 4 455 165 A1**

(12) **EUROPEAN PATENT APPLICATION**
published in accordance with Art. 153(4) EPC

(43) Date of publication:
**30.10.2024 Bulletin 2024/44**

(21) Application number: **22909417.2**

(22) Date of filing: **23.09.2022**

(51) International Patent Classification (IPC):
*C07K 16/46* (2006.01)        *C12N 15/13* (2006.01)
*A61K 39/395* (2006.01)        *C07K 16/00* (2006.01)
*A61P 27/16* (2006.01)

(86) International application number:
**PCT/CN2022/120996**

(87) International publication number:
**WO 2023/116099 (29.06.2023 Gazette 2023/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.12.2021 CN 202111585813**

(71) Applicant: **EXCELMAB INC.**
**Huangpu District**
**Guangzhou**
**Guangdong 510000 (CN)**

(72) Inventors:
• **ZHANG, Wenjun**
**Guangzhou, Guangdong 510000 (CN)**

• **LI, Feng**
**Guangzhou, Guangdong 510000 (CN)**
• **LIU, Min**
**Guangzhou, Guangdong 510000 (CN)**
• **PAN, Zhihao**
**Guangzhou, Guangdong 510000 (CN)**
• **QIN, Luying**
**Guangzhou, Guangdong 510000 (CN)**
• **CAO, Guangcan**
**Guangzhou, Guangdong 510000 (CN)**

(74) Representative: **Von Rohr Patentanwälte**
**Partnerschaft mbB**
**Rüttenscheider Straße 62**
**45130 Essen (DE)**

Remarks:
The complete document including Reference Table(s) and the Sequence Listing(s) can be downloaded from the EPO website

(54) **BISPECIFIC ANTIBODY AND USE THEREOF**

(57)      Provided herein is a bispecific antibody and the use thereof. The bispecific antibody comprises an antibody or antibody fragment; the antibody or the antibody fragment is a bispecific antibody or an antibody fragment comprising a first heavy chain, a first light chain, a second heavy chain, and a second light chain; the first Fab region comprises the first heavy chain and the first light chain; the second Fab region comprises the second heavy chain and the second light chain; and a variety of mutations are introduced in the first Fab region and the second Fab region. By means of performing amino acid size and charge mutations on amino acid residues at a heavy chain CH1 and light chain CL interface of the antibody or the antibody fragment, the correct pairing of heavy/light chains targeting different antigens is enhanced, the binding activity to specific antigens is maintained, mismatches are reduced, the binding specificity is improved, and the yield of a target product is improved.

FIG. 1

EP 4 455 165 A1

**Description**

**Cross Reference to Related Applications**

The present invention claims priority to Chinese Patent Application No. CN202111585813.8, filed with the China National Intellectual Property Administration on December 20, 2021, entitled "BISPECIFIC ANTIBODIES AND USE THEREOF", the entirety of which is incorporated herein by reference.

**Technical Field**

The present invention belongs to the technical field of protein engineering and relates to methods for intramolecular or intermolecular modification of proteins. Specifically, the present invention provides a method for mutating amino acids at the interface between a heavy chain CH1 and a light chain CL of a bispecific antibody containing two different light chains. These alterations involve changes in the size and charge of the amino acids to prevent incorrect pairing and thereby improve binding specificity. The present invention particularly relates to bispecific antibodies and the use thereof.

**Background in the Art**

Bispecific antibodies, which are capable of simultaneously engaging two different antigenic epitopes, exhibit unique biological activities and can produce superior additive or synergistic effects compared to monoclonal antibodies. They have emerged as a significant area of research and development within antibody engineering and oncology immunotherapy. In some previous studies, many attempts have been made in antibodies engineering (Spiess et al., Molecular Immunology, 67(2), pp. 95-106 (2015)) concerning new formats of bispecific antibodies (e.g., DVD-Ig, Cross-Mab, BiTE, and the like). However, these formats may have multiple limitations in terms of stability, solubility, half-life, immunogenicity, etc.

In the field of bispecific antibody development, the IgG-type bispecific antibody is a prevalent format, which structurally comprises a half antibody binding antigen A and the other half antibody targeting antigen B, exhibiting a size and shape analogous to that of a natural IgG. In the preparation of an IgG-type bispecific antibody, it is necessary to simultaneously express two different heavy chains and two different light chains. These chains must form two different Fabs capable of recognizing two different antigens through precise pairing. However, the process results in the production of more than ten different products due to mispairing caused by the homologous heavy chain binding and heterologous light chain binding. Only the correctly paired complex of heavy and light chains yields the desired bispecific antibody. Although target bispecific antibodies can be obtained through downstream purification, the process is often highly inefficient and restricts large-scale production. Therefore, the main challenges in the preparation of bispecific antibodies involve addressing the random assembly of different chains, which includes mispairing of homologous heavy chains, mispairing of heterologous light chains, and contamination with non-target products.

"Knob-into-hole" is the first solution to the problem of heavy chain pairing (Ridgway et al., Protein Engineering, 9(7), pp. 617-21 (1996); Merchant et al., Nature Biotechnology, 16(7), pp. 677-681 (1998)). Furthermore, techniques such as electrostatic steering (Gunasekaran et al., Journal of Biological Chemistry, 285(25), pp. 19637-19646 (2010)) and negative design (Kreudenstein et al., mAbs, 5(5), pp. 646-654 (2013); Leaver-Fay et al., Structure, 24(4), pp. 641-651 (2016)) are also employed to address heavy chain mispairing. Although these strategies effectively mitigate homologous heavy chain mispairing, they do not resolve the issue of heterologous heavy-light chain pairing.

Therefore, providing a method to reduce mispairing between heavy and light chains and improve the yield of the desired product is an imperative challenge to address.

**Summary**

The present invention provides an antibody or an antibody fragment, wherein the antibody or antibody fragment is a bispecific antibody or antibody fragment comprising a first heavy chain and a first light chain, as well as a second heavy chain and a second light chain;

The first Fab region comprises the first heavy chain and the first light chain;

The second Fab region comprises the second heavy chain and the second light chain;

At least one mutation, or a combination of at least two mutations, from the following set is introduced into the first Fab region of the antibody or antibody fragment:

a) S186A/S186T/S186E/S186Q/S186M/S186D/S186K/S186V/S186W/S18 6Y in a heavy chain constant domain CH1; and
T178M/T178Y/T178L/T178L/T178A/T178V/T178S/T178R/T178E in a light chain constant domain CL;

b) S188Y/S188M/S188T/S188F/S188V/S188L/S188I/S188R in the heavy chain constant domain CH1; and
S176M/S176L/S176Y/S176A/S176I/S176E/S176T/S176W/S176H/S176V in the light chain constant domain CL;

c) V190T/V190I/V190W/V190M/V190Y/V190F/V190L/V190Q/V190S in the heavy chain constant domain CH1; and
S174F/S174N/S174M/S174A/S174T/S174W/S174R/S174C/S174K/S174H/ S174Y/S174L in the light chain constant domain CL;

At least one mutation, or a combination of at least two mutations, from the following set is introduced into the second Fab region of the antibody or antibody fragment:

a) S186A/S186T/S186E/S186Q/S186M/S186D/S186K/S186V/S186W/S18 6Y in a heavy chain constant domain CH1; and
T178M/T178Y/T178L/T178I/T178A/T178V/T178S/T178R/T178E in a light chain constant domain CL;

b) S188Y/S188M/S188T/S188F/S188V/S188L/S188I/S188R in the heavy chain constant domain CH1; and
S176M/S176L/S176Y/S176A/S176I/S176E/S176T/S176W/S176H/S176V in the light chain constant domain CL;

c) V190T/V190I/V190W/V190M/V190Y/V190F/V190L/V190Q/V190S in the heavy chain constant domain CH1; and
S174F/S174N/S174M/S174A/S174T/S174W/S174R/S174C/S174K/S174H/ S174Y/S174L in the light chain constant domain CL.

In some embodiments, any one mutation of the following sets is introduced into the first Fab region of the antibody or antibody fragment:

a) S186T in the heavy chain constant domain CH1 and T178A in the light chain constant domain CL;

b) S188Y in the heavy chain constant domain CH1 and S176E in the light chain constant domain CL;

c) V190F in the heavy chain constant domain CH1 and S174A in the light chain constant domain CL;

or

a) S186V in the heavy chain constant domain CH1 and T178L in the light chain constant domain CL;

b) S188L in the heavy chain constant domain CH1 and S176W in the light chain constant domain CL;

c) V190M in the heavy chain constant domain CH1 and S174R in the light chain constant domain CL;

or

a) S186W in the heavy chain constant domain CH1 and T178M in the light chain constant domain CL;

b) S188F in the heavy chain constant domain CH1 and S176V in the light chain constant domain CL;

c) V190T in the heavy chain constant domain CH1 and S174K in the light chain constant domain CL;

or

a) S186Y in the heavy chain constant domain CH1;

b) S188M in the heavy chain constant domain CH1 and S176V in the light chain constant domain CL;

c) V190F in the heavy chain constant domain CH1;

Preferably, any one mutation of the following sets is introduced into the second Fab region of the antibody or antibody fragment:

a) S186M in the heavy chain constant domain CH1 and T178V in the light chain constant domain CL;

b) S188I in the heavy chain constant domain CH1 and S176H in the light chain constant domain CL;

c) V190W in the heavy chain constant domain CH1 and S174R in the light chain constant domain CL;

or

a) S186T in the heavy chain constant domain CH1 and T178V in the light chain constant domain CL;

b) S188R in the heavy chain constant domain CH1;

c) V190F in the heavy chain constant domain CH1;

or

a) S186K in the heavy chain constant domain CH1 and T178E in the light chain constant domain CL;

b) S188L in the heavy chain constant domain CH1 and S176M in the light chain constant domain CL;

c) S174Y in the light chain constant domain CL;

or

a) S186M in the heavy chain constant domain CH1 and T178V in the light chain constant domain CL;

b) S188I in the heavy chain constant domain CH1 and S176H in the light chain constant domain CL;

c) V190W in the heavy chain constant domain CH1 and S174R in the light chain constant domain CL.

In some embodiments, an A178D mutation is further introduced into the light chain constant domain CL of the first Fab region, while an I188D mutation is further introduced into the heavy chain constant domain CH1 of the second Fab region.

In some embodiments, an L178K mutation is further introduced into the light chain constant domain CL of the first Fab region, while a T192K mutation is further introduced into the heavy chain constant domain CH1 of the second Fab region.

In some embodiments, the mutations introduced within the first Fab region and the second Fab region of the antibody or antibody fragment may be the same or different.

The present invention further provides a bispecific antibody, wherein the bispecific antibody comprises an antibody or antibody fragment as described in any of the preceding paragraphs.

In some embodiments, the light chain constant domain CL of the bispecific antibody is each independently selected from any one or more of the following sequences: SEQ ID NO: 3, SEQ ID NO: 7, SEQ ID NO: 11, SEQ ID NO: 15, SEQ ID NO: 19, SEQ ID NO: 23, SEQ ID NO: 27, SEQ ID NO: 31, SEQ ID NO: 35, SEQ ID NO: 39, SEQ ID NO: 43, and SEQ ID NO: 47, SEQ ID NO: 51, SEQ ID NO: 55, SEQ ID NO: 59, SEQ ID NO: 63, SEQ ID NO: 67, SEQ ID NO: 71, SEQ ID NO: 75, SEQ ID NO: 79, SEQ ID NO: 83, SEQ ID NO: 87, SEQ ID NO: 91, SEQ ID NO: 95, SEQ ID NO: 99, SEQ ID NO: 103, SEQ ID NO: 107, SEQ ID NO: 111, or SEQ ID NO: 115.

In some embodiments, the heavy chain constant domain CH1 of the bispecific antibody is each independently selected from any one or more of the following sequences: SEQ ID NO: 1, SEQ ID NO: 5, SEQ ID NO: 9, SEQ ID NO: 13, SEQ ID NO: 17, SEQ ID NO: 21, SEQ ID NO: 25, SEQ ID NO: 29, SEQ ID NO: 33, SEQ ID NO: 37, SEQ ID NO: 41, SEQ ID NO: 45, SEQ ID NO: 49, SEQ ID NO: 53, SEQ ID NO: 57, SEQ ID NO: 61, SEQ ID NO: 65, SEQ ID NO: 69, SEQ ID NO: 73, SEQ ID NO: 77, SEQ ID NO: 81, SEQ ID NO: 85, SEQ ID NO: 89, SEQ ID NO: 93, SEQ ID NO:

97, SEQ ID NO: 101, SEQ ID NO: 105, SEQ ID NO: 109, or SEQ ID NO: 113.

In some embodiments, the first Fab region and the second Fab region of the bispecific antibody are each independently selected from any one or more of the following sequences:

a light chain constant domain CL of SEQ ID NO: 35 and a heavy chain constant domain CH1 of SEQ ID NO: 33; or

a light chain constant domain CL of SEQ ID NO: 79 and a heavy chain constant domain CH1 of SEQ ID NO: 77; or

a light chain constant domain CL of SEQ ID NO: 55 and a heavy chain constant domain CH1 of SEQ ID NO: 53; or

a light chain constant domain CL of SEQ ID NO: 115 and a heavy chain constant domain CH1 of SEQ ID NO: 113; or

a light chain constant domain CL of SEQ ID NO: 83 and a heavy chain constant domain CH1 of SEQ ID NO: 81; or

a light chain constant domain CL of SEQ ID NO: 91 and a heavy chain constant domain CH1 of SEQ ID NO: 89; or

a light chain constant domain CL of SEQ ID NO: 107 and a heavy chain constant domain CH1 of SEQ ID NO: 105; or

a light chain constant domain CL of SEQ ID NO: 79 and a heavy chain constant domain CH1 of SEQ ID NO: 77.

In some embodiments, the first Fab region and the second Fab region of the bispecific antibody bind to different antigens or two different epitopes of the same antigen.

In some embodiments, the antigen is independently selected from any one or a combination of two of CD2, CD3, CD3E, CD4, CD11, CD11a, CD14, CD16, CD18, CD19, CD20, CD22, CD23, CD25, CD28, CD29, CD30, CD32a, CD32b, CD33, CD38, CD40, CD40L, CD52, CD54, CD56, CD64, CD80, CD147, GD3, IL-1α, IL-1β, IL-1R, IL-2, IL-2R, IL-4, IL-5, IL-5R, IL-6, IL-6R, IL-8, IL-9, IL-12, IL-13, IL-15, IL-17, IL-17R, IL-18, IL-23, interferon α, interferon β, interferon γ, TNF-α, TNF-β, TNF-R1, TNF-RII, FasL, CD27L, CD30L, 4-1BBL, TRAIL, RANKL, TWEAK, APRIL, BAFF, LIGHT, VEG1, OX40L, TRAIL receptor-1, adenosine receptor, lymphotoxin-β receptor, TACI, BAFF-R, EPO; LFA-3, ICAM-1, ICAM-3, EpCAM, integrin β1, integrin β2, integrin α4/β7, integrin α2, integrin α3, integrin α4, integrin α5, integrin α6, integrin αv, integrin αVβ3, FGFR-3, keratinocyte growth factor, VLA-1, VLA-4, L-selectin, anti-Id, E-selectin, HLA, HLA-DR, CTLA-4, T cell receptor, B7-1, B7-2, VNR integrin, TGFβ1, TGFβ2, eotaxin 1, Blys, complement C5, IgE, factor VII, CD64, CBL, NCA 90, EGFR, Her1, Her2/neu, Her3, Her4, tissue factor, endothelin receptor, VLA-4, hapten NP-cap, NIP-cap, E-selectin, digoxin, placental alkaline phosphatase, testicular PLAP-like alkaline phosphatase, transferrin receptor, carcinoembryonic antigen, CEACAM5, HMFG1, PEM, mucin MUC1, MUC18, heparinase I, human cardiac myosin, tumor-associated glycoprotein-72, tumor-associated antigen CA125, prostate-specific membrane antigen, high molecular weight melanoma-associated antigen, carcinoma-associated antigen,Glycoprotein IIb/IIIa, tumor-associated antigen expressing Lewis Y-associated carbohydrate, human cytomegalovirus gH envelope glycoprotein, HIV gp120, HCMV, respiratory syncytial virus RSV F, RSVF Fgp, cytokeratin tumor-associated antigen, Hep B gp120, CMV, gpIIb/IIIa, HIV IIIB gp120V3 loop, respiratory syncytial virus Fgp, herpes simplex virus gD glycoprotein, HSV gB glycoprotein, HCMV gB envelope glycoprotein, *Clostridium perfringens* toxin, CD133, CD138, OX40, GITR, PD-1, PDL1, PD-L2, CTLA-4, KIR, LAG-3, TCRα, TCRβ, TCRγ, TCRδ, VEGF, EGF, VEGFR, EGFR, EpCAM, mesothelin, glypicans, Erb1, Erb2, B7-H3, ICOS, BMP1, BMP2, BMP3B, BMP4, CSF1, GM-CSF, FGF1, FGF2, FGF3, FGF4, PDGFR, TIGIT, CS1, TWEAK, CCL1, CCL2, CCL3, CCL13, CXCL1, CXCL2, CXCL3, IP-10, fucosyl-GM1, IGF1, IGF2, IGF1R, IGF2R, RANK ligand, DLL-4, GM-CSFR, ADAMS, myostatin, PCSK9, CXCR4, MIF, or PEG2.

In some embodiments, the first Fab region of the bispecific antibody binds to any one of CD3, CD3E, CD16, CD28, CD47, CD27, CD40, OX40, GITR, ICOS, 4-1BB, PD-1, PD-L1, GITR, TIGIT, TIM3, LAG3, B7H3, CTLA4, VISTA, BTLA, HVEM, NKG2A, or NECL.

In some embodiments, the second Fab region of the bispecific antibody binds to any one of CD2, CD4, CD11, CD11a, CD14, CD18, CD19, CD20, CD22, CD23, CD25, CD29, CD30, CD32a, CD32b, CD33, CD38, CD52, CD54, CD56, CD64, CD80, CD147, GD3, IL-1α, IL-1β, IL-1R, IL-2, IL-2R, IL-4, IL-5, IL-5R, IL-6, IL-6R, IL-8, IL-9, IL-12, IL-13, IL-15, IL-17, IL-17R, IL-18, IL-23, interferon α, interferon β, interferon γ, TNF-α, TNF-β, TNF-R1, TNF-RII, TACI, BAFF-R, EPO; LFA-3, GPC-3, ICAM-1, ICAM-3, complement C5, IgE, factor VII, CD64, CBL, NCA 90, Her1, Her2, Her3, Her4, carcinoembryonic antigen, tumor-associated glycoprotein-72, tumor-associated antigen CA125, prostate-specific membrane antigen, high molecular weight melanoma-associated antigen, carcinoma-associated antigen, or tumor-associated antigen expressing Lewis Y-associated carbohydrate.

In some embodiments, the antigen to which the first Fab region of the bispecific antibody binds is CD3, and the antigen to which the second Fab region binds is GPC3.

In some embodiments, the bispecific antibody comprises a first heavy chain and a first light chain binding to CD3, and a second heavy chain and a second light chain binding to GPC3.

In some embodiments, the bispecific antibody is selected from any one of the following amino acid sequences:

the first heavy chain has an amino acid sequence set forth in SEQ ID NO: 129, and the first light chain has an amino acid sequence set forth in SEQ ID NO: 127; the second heavy chain has an amino acid sequence set forth in SEQ ID NO: 133, and the second light chain has an amino acid sequence set forth in SEQ ID NO: 131; or

the first heavy chain has an amino acid sequence set forth in SEQ ID NO: 145, and the first light chain has an amino acid sequence set forth in SEQ ID NO: 143; the second heavy chain has an amino acid sequence set forth in SEQ ID NO: 149, and the second light chain has an amino acid sequence set forth in SEQ ID NO: 147; or

the first heavy chain has an amino acid sequence set forth in SEQ ID NO: 137, and the first light chain has an amino acid sequence set forth in SEQ ID NO: 135; the second heavy chain has an amino acid sequence set forth in SEQ ID NO: 141, and the second light chain has an amino acid sequence set forth in SEQ ID NO: 139; or

the first heavy chain has an amino acid sequence set forth in SEQ ID NO: 153, and the first light chain has an amino acid sequence set forth in SEQ ID NO: 151; the second heavy chain has an amino acid sequence set forth in SEQ ID NO: 157, and the second light chain has an amino acid sequence set forth in SEQ ID NO: 155.

In some embodiments, the bispecific antibody is derived from IgG, IgA, IgM, IgE, or IgD.

In some embodiments, the bispecific antibody is derived from IgG1, IgG2, IgG3, or IgG4.

The present invention provides a nucleic acid molecule, the nucleic acid molecule encoding an antibody or antibody fragment, and/or a bispecific antibody, as described in any of the preceding embodiments.

The present invention further provides an expression vector comprising the nucleic acid molecule described herein.

The present invention further provides a recombinant cell that expresses an antibody or antibody fragment, and/or the bispecific antibodies in any one of claims 4~5, as described in any of the preceding embodiments.

In some embodiments, the nucleic acid molecule is integrated into the genome of the recombinant cell.

In some embodiments, the recombinant cell comprises the expression vector.

The present invention further provides a method for preparing an antibody or antibody fragment as described in any of the preceding embodiments, comprising the steps:

inserting a nucleic acid molecule encoding the antibody or antibody fragment into a plasmid, transferring the plasmid into competent cells, and subsequently selecting monoclonal cells for screening;

purifying the expression vector from the screened positive clone, transferring the vector into recipient cells, and obtaining recombinant cells through screening;

culturing the recombinant cells, collecting the culture supernatant, and isolating and purifying the antibody or antibody fragment from the collected supernatant.

The present invention further provides an antibody conjugate, which comprises an antibody or antibody fragment, and/or a bispecific antibody, as described in any of the preceding embodiments, and a conjugated marker.

In some embodiments, the conjugated marker comprises any one or a combination of at least two of a cytotoxin, a radioisotope, a fluorescent marker, a luminescent substance, a chromogenic substance, or an enzyme.

The present invention further provides a pharmaceutical composition, which comprises any one or a combination of at least two of an antibody or antibody fragment, and/or a bispecific antibody or the antibody conjugate, as described in any of the preceding embodiments.

In some embodiments, the pharmaceutical composition further comprises any one or more, or a combination of at least two of a pharmaceutically acceptable carrier, a surfactant, a diluent, or an excipient, either individually or in combination.

The present invention further provides the use of any one or more, or a combination of at least two of the antibody or antibody fragment, and/or a bispecific antibody, the antibody conjugate, or the pharmaceutical composition, as described in any of the preceding embodiments, in the preparation of a medicament for the treatment of a disease.

In some embodiments, the disease includes cancer.

The present invention further provides use of any one or a combination of at least two of the antibody or antibody fragment, and/or a bispecific antibody, as described in any of the preceding embodiments, the antibody conjugate, or the pharmaceutical composition, for the treatment of a disease.

In some embodiments, the disease includes cancer.

The present invention further provides a method for treating a disease, the method comprising: administering any one or a combination of at least two of the antibody or antibody fragment, and/or a bispecific antibody, the antibody conjugate, or the pharmaceutical composition, as described in any of the preceding embodiments, to a subject in need thereof.

In some embodiments, the disease includes cancer.

**Brief Description of Drawings**

FIG. 1 illustrates a schematic structural diagram of a binding interface of the E-strand in a CH1-CL domain.

FIG. 2 illustrates a schematic structural diagram of a bispecific antibody, wherein Figure I is a schematic structural diagram of two Crossmab bispecific antibodies, Figure II is a schematic structural diagram of a traditional triple-chain bispecific antibody, and Figure III is a schematic structural diagram of a modified bispecific antibody in the present invention.

FIG. 3A shows the results of reducing and non-reducing SDS-PAGE electrophoresis of $WT_0$; FIG. 3B shows the results of reducing and non-reducing SDS-PAGE electrophoresis of CM-C; FIG. 3C shows the results of reducing and non-reducing SDS-PAGE electrophoresis of CM-G; FIG. 3D shows the results of reducing and non-reducing SDS-PAGE electrophoresis of WT; FIG. 3E shows the results of reducing and non-reducing SDS-PAGE electrophoresis of MutA and MutB; FIG. 3F shows the results of reducing and non-reducing SDS-PAGE electrophoresis of MutA+D-D and MutB+K-K.

FIG. 4A shows the results of the analysis of MutA, MutB, and the negative controls $WT_0$, CM-C, and CM-G against GPC3 by ELISA.

FIG. 4B shows the results of the analysis of MutA, MutB, and the negative controls $WT_0$, CM-C, and CM-G against CD3 by ELISA.

FIG. 4C shows the results of the analysis of MutA+D-D, MutB+K-K, and the negative controls $WT_0$, CM-C, and CM-G against GPC3 by ELISA.

FIG. 4D shows the results of the analysis of MutA+D-D, MutB+K-K, and the negative controls $WT_0$, CM-C, and CM-G against CD3 by ELISA.

FIG. 5A shows the results of the analysis of MutA, MutB, the triple-chain positive antibody WT, and hIgG against GPC3 by ELISA.

FIG. 5B shows the results of the analysis of MutA, MutB, the triple-chain positive antibody WT, and hIgG against CD3 by ELISA.

FIG. 5C shows the results of the analysis of MutA+D-D, MutB+K-K, the triple-chain positive antibody WT, and hIgG against GPC3 by ELISA.

FIG. 5D shows the results of the analysis of MutA+D-D, MutB+K-K, the triple-chain positive antibody WT, and hIgG against CD3 by ELISA.

FIG. 6 is a diagram illustrating the analysis of the GPC3-binding activity of MutA, MutB, MutA+D-D, MutB+K-K, and the triple-chain positive antibody WT by flow cytometry.

FIG. 7A is a diagram showing the results of the T cell activation activity of MutA, MutB, and the negative controls $WT_0$, CM-C, and CM-G.

FIG. 7B is a diagram showing the results of the T cell activation activity of MutA+D-D, MutB+K-K, and negative controls $WT_0$, CM-C, and CM-G.

FIG. 8A is a diagram showing the results of the T cell activation activity of MutA, MutB, and the triple-chain positive antibody WT.

FIG. 8B is a diagram showing the results of the T cell activation activity of MutA+D-D, MutB+K-K, and the triple-chain positive antibody WT.

FIG. 9A is a diagram showing the results of the tumor cell-killing activity of MutA+D-D and the negative controls $WT_0$, CM-C, and CM-G.

FIG. 9B is a diagram showing the results of the tumor cell-killing activity of MutA, MutB, and the triple-chain positive antibody WT.

FIG. 9C is a diagram showing the results of the tumor cell-killing activity of MutA+D-D, MutB+K-K, and the triple-chain positive antibody WT.

**Detailed Description of Embodiments**

The present invention is further illustrated below with reference to specific embodiments, examples, and the accompanying drawings to elucidate the technical features employed in the present invention and effects thereof. It is understood that the embodiments described herein are for the purpose of illustration only and are not intended to limit the scope of the present invention.

Embodiments and examples, for which specific techniques or conditions are not specified, are carried out in accordance with techniques or conditions described in the literature in the art or in accordance with the product instructions. Reagents or instruments used that are not specified with manufacturers are all conventional products that can be purchased through standard commercial channels.

Unless otherwise defined herein, scientific and technical terms and their abbreviations used in conjunction with the present invention shall have meanings commonly understood by those skilled in the art to which the present invention pertains. Some of the terms and abbreviations used herein are listed below.

Antibody, Ab;

Immunoglobulin, Ig;

Heavy chain, HC;

Light chain, LC;

Heavy chain variable domain, VH;

Heavy chain constant domain, CH;

Light chain variable domain, VL;

Light chain constant domain, CL;

Antigen binding fragment, Fab;

Hinge region;

Fc fragment: fragment crystallizable region, Fc region;

Monoclonal antibodies, mAbs;

Antibody-dependent cell-mediated cytotoxicity, ADCC;

Complement dependent cytotoxicity, CDC;

Natural killing cells, NK cells;

Bispecific antibody, BsAb;

T cell receptor, TCR;

Major histocompatibility complex, MHC;

Complementarity determining region, CDR, which refers to an antigen complementary binding region of an antibody;

Immunoreceptor tyrosine-based activation motif, ITAM;

Single-chain variable fragment, scFv;

Adoptive cellular immunotherapy, ACI;

Lymphokine-activated killer cell, LAK cell;

Tumor-infiltrating lymphocyte, TIL cell;

Cytokine-induced killer cell, CIK cell.

Unless otherwise defined, the relevant terms used in the present invention have the same meanings as those commonly understood in this technical field. The operation steps of tests such as molecular cloning, cell culturing, protein purification, immunological experiments, microbiology, and animal models described in the present invention are conventional steps widely used in this field. Unless otherwise required by the context, singular terms in the present invention include pluralities and plural meanings include a singular meaning. Unless otherwise specified, nucleotide sequences described in the present invention are arranged and written from left to right in the direction from the 5' end to the 3' end. Unless otherwise specified, amino acid sequences described in the present invention are arranged and written from left to right in the direction from the amino terminus (N-terminus) to the carboxyl terminus (C-terminus). Three-letter abbreviations for amino acids and single-letter abbreviations for nucleotides mentioned in the present invention are in the form generally accepted in this technical field, and single-letter abbreviations for amino acids are in the form recommended by the IUPAC-IUB Biochemical Nomenclature Commission.

As used herein, the term "amino acid" refers to one of the 20 naturally occurring amino acids or any non-natural analog that may be present at a specifically defined position. The term "amino acid mutations" described in the present invention refers to substitutions, insertions, deletions, and modifications of amino acids in polypeptide sequences, as well as any combination of substitutions, insertions, deletions, and modifications of amino acids. A preferred amino acid modification herein is a substitution. In the present invention, the "amino acid substitution" or "substitution" refers to replacement of an amino acid at a specific position in a parental polypeptide sequence with another amino acid. For example, a C220S substitution refers to a variant polypeptide in which amino acid (cysteine) at position 220 of the polypeptide has been replaced by an amino acid (serine). Amino acid mutations can be achieved by molecular cloning or chemical methods, the molecular cloning method including PCR, site-directed mutagenesis, whole-gene synthesis, and the like.

As used herein, the terms "protein", "peptide chain", and "polypeptide chain" refer to molecules in which two or more amino acids are linked by a peptide bond, including native proteins, artificial proteins, protein fragments, mutant proteins, fusion proteins, and the like.

The term "domain" refers to a specific structural region with an independent function in a biological macromolecule, where the domain has an independent tertiary structure, and its function does not depend on the rest of the biological macromolecule. A domain in the present invention specifically refers to such a region in a protein, such as a heavy chain variable domain (VH domain) or a light chain variable domain (VL domain), and the domains can be combined with each other to form a large domain.

As used herein, the term "antibody" refers to an immunoglobulin molecule containing at least one antigen recognition site and capable of specifically binding to an antigen. Herein, the term "antigen" refers to a substance that can induce an immune response *in vivo* and binds specifically to an antibody, such as a protein, a polypeptide, a peptide, a carbohydrate, a polynucleotide, a lipid, a hapten, or a combination of the above substances. The binding of antibodies to antigens is mediated by the interactions formed between them, including hydrogen bonds, van der Waals forces, ionic bonds, and hydrophobic bonds. A region on the surface of an antigen that is bound to an antibody is called an "antigenic determinant" or "epitope", and in general, each antigen has a plurality of determinants. The term "antibody" mentioned in the present invention includes monoclonal antibodies (including full-length monoclonal antibodies), polyclonal antibodies, antibody fragments, multispecific antibodies containing at least two different epitope-binding domains (e.g.,

bispecific antibodies), human antibodies, humanized antibodies, post-translationally modified antibodies, camel antibodies, chimeric antibodies, fusion proteins containing antibody/antigenic determinants, and any other modified immunoglobulin molecules containing antigen recognition sites, as long as these antibodies exhibit the desired biological activity. Specifically, antibodies include immunoglobulin molecules and immunoactive fragments of immunoglobulin molecules, i.e., molecules comprising at least one antigen-binding site.

As used herein, the term "bispecific antibody" refers generally to an artificially designed or artificially synthesized antibody capable of simultaneously targeting two antigens or two different epitopes of one antigen.

As used herein, the term "tetra-chain bispecific antibody" refers generally to a bispecific antibody consisting of two heavy chains and two light chains. Here, the first heavy chain and the second heavy chain may be the same or different. The first light chain and the second light chain may be the same or different. The first heavy chain and the first light chain constitute a domain binding to one antigen or more antigens, and the second heavy chain and the second light chain constitute a domain binding to one antigen or more antigens. For example, the antibody described herein is a bispecific antibody consisting of two different heavy chains and two different light chains. This bispecific antibody contains two Fab regions that can bind to different antigens or to different epitopes of the same antigen, respectively. Here, the first Fab region consists of a VH domain and a CH1 domain of the first heavy chain and a VL domain and a CL domain of the first light chain; and the second Fab region consists of a VH domain and a CH1 domain of the second heavy chain and a VL domain and a CL domain of the second light chain.

As used herein, the term "Fab", "Fab region", "Fab fragment", or "Fab molecule" refer to an antigen-binding fragment containing a VH domain and a CH1 domain of an immunoglobulin heavy chain, as well as a VL domain and a CL domain of a light chain, where the first constant domain CH1 of the heavy chain binds to the constant domain CL of the light chain, and the variable domain VH of the heavy chain binds to the variable domain VL of the light chain.

As used herein, the term "Fc", "Fc region", "Fc fragment", or "Fc molecule" is an effector region of an antibody that can induce, e.g., CDC, ADCC, ADCP, and cytokine release. Natural antibody Fc is usually formed by binding of two identical protein fragments, where the protein fragment contains two or three immunoglobulin constant domains. The Fc described in the present invention includes natural Fc and mutated Fc. Although the boundaries of the Fc region may vary, the human IgG heavy chain Fc region is usually defined to contain residues from C226 or P230 to its carboxyl terminus. Under experimental conditions, immunoglobulin monomers are digested by papain to generate Fab and Fc fragments, respectively.

As used herein, the term "hinge" or "hinge region" refers to a flexible polypeptide containing amino acids between the first and second constant domains (CH1 and CH2) of an antibody.

Unless otherwise noted, amino acids of antibodies described in the present invention are numbered using the numbering scheme elaborated by Kabat *et al.* in 1991, i.e., "Kabat index" or "Kabat numbering" (Kabat, E. A. et al., Sequences of Proteins of Immunological Interest, 5th ed., NIH Publication No. 91-3242, Bethesda, MD.:1991).

As used herein, the term "antigen-binding site" refers to one or more amino acid residues at which an antigen-binding molecule directly interacts with an antigen, where the antigen-binding site of an antibody consists of an antigen-complementarity determining region (CDR), a natural immunoglobulin molecule usually contains two antigen-binding sites and a Fab molecule usually contains one antigen-binding site.

As used herein, the term "T cell activation" refers to one or more immune responses of T lymphocytes, especially killer T lymphocytes, including proliferation, differentiation, release of cytokines, secretion of killer effector molecules, cell killing, and the like.

As used herein, the term "$EC_{50}$" stands for a concentration of 50% of maximal effect, which refers to the corresponding antibody concentration that elicits 50% of the maximum effect.

The term "specific binding" used herein refers to a non-random binding reaction between two molecules, such as a reaction between an antibody and its targeted antigen. In some embodiments, an antibody specifically binding to an antigen (or an antibody having specificity to an antigen) refers to an antibody binding to the antigen with an affinity (KD) of less than about $10^{-5}$ M, e.g., less than about $10^{-6}$ M, $10^{-7}$ M, $10^{-8}$ M, $10^{-9}$ M, $10^{-10}$ M, or less. In some embodiments of the present invention, the term "targeting" refers to specific binding.

The term "KD" used herein refers to the dissociation equilibrium constant of a particular antibody-antigen interaction, for describing the binding affinity between the antibody and the antigen. A lower equilibrium dissociation constant indicates a tighter antibody-antigen binding and a higher affinity between the antibody and the antigen. Typically, an antibody binds to an antigen at an equilibrium dissociation constant (KD) of less than about $10^{-5}$ M, e.g., less than about $10^{-6}$ M, $10^{-7}$ M, $10^{-8}$ M, $10^{-9}$ M, $10^{-10}$ M, or less.

As used herein, the term "single-chain variable fragment" or "scFv" refers to a fusion protein comprising a heavy chain variable domain VH and a light chain variable domain VL of an immunoglobulin, including different combined forms in which VH is at N-terminus and in which VL is at N-terminus, which can be prepared by using a conventional molecular cloning method for construction of recombinant proteins (Sambrook JF, E. F. et al., Molecular cloning: a laboratory manual. 4th ed. Cold Spring Harbor Laboratory Press, Cold Spring Harbor, New York: 2012).

As used herein, the term "humanized antibody" refers to an antibody or antibody fragment obtained by replacing some or all of the CDR regions of a human immunoglobulin (acceptor antibody) with CDR regions of a non-human antibody (donor antibody), where the donor antibody may be a non-human (e.g., mouse, rat, or rabbit) antibody with the expected specificity, affinity, or reactivity. In addition, some amino acid residues in the framework region (FR) of the acceptor antibody may also be replaced by amino acid residues of the corresponding non-human antibody, or by amino acid residues of other antibodies, to further improve or optimize one or more characteristics of the antibody.

The term "host cell" refers to a cell incorporated with an exogenous nucleic acid and its progeny, which may be transformed or transfected with a nucleotide encoding a polypeptide so as to express the exogenous polypeptide. The host cells described in the present invention include but are not limited to, CHO cells (Chinese hamster ovary cells), HEK293 cells (Human Embryonic Kidney cells 293), BHK cells (Baby Hamster Kidney cells), myeloma cells, yeast, insect cells, or prokaryotic cells such as *Escherichia coli.* It should be noted that the term "host cell" described in the present invention includes not only a cell incorporated with an exogenous nucleic acid but also progeny of the cell, which still falls within the scope of the term described in the present invention, although mutations may occur in the progeny cells during cell division.

As used herein, an "effective amount" may refer to the amount of a composition described herein or the amount of a pharmaceutical formulation described herein, which will elicit the desired biological or medical response of a tissue, a system, an animal, a plant, a protozoan, a bacterium, yeast, or a human that is being sought by a researcher, a veterinarian, a medical doctor, or other clinician.

As used herein, the term "subject" refers to a vertebrate, optionally a mammal, optionally a human. Mammals include, but are not limited to, rats, apes, humans, domestic animals, competitive animals, and pets. Tissues, cells, and their progeny of biological entities obtained *in vivo* or cultured *in vitro* are also included.

It should be understood that the terms used herein are used only for the purpose of describing the context of the present invention, such as embodiments and examples, and are not intended to limit the scope of the invention.

Some embodiments of the present invention provide an antibody or antibody fragment, wherein the antibody or antibody fragment is a bispecific antibody or antibody fragment comprising a first heavy chain and a first light chain, as well as a second heavy chain and a second light chain;

The first Fab region comprises the first heavy chain and the first light chain;

The second Fab region comprises the second heavy chain and the second light chain;

At least one mutation, or a combination of at least two mutations, from the following set is introduced into the first Fab region of the antibody or antibody fragment:

    a) S186A/S186T/S186E/S186Q/S186M/S186D/S186K/S186V/S186W/S18 6Y in a heavy chain constant domain CH1; and
    T178M/T178Y/T178L/T178I/T178A/T178V/T178S/T178R/T178E in a light chain constant domain CL;

    b) S188Y/S188M/S188T/S188F/S188V/S188L/S188I/S188R in the heavy chain constant domain CH1; and
    S176M/S176L/S176Y/S176A/S176I/S176E/S176T/S176W/S176H/S176V in the light chain constant domain CL;

    c) V190T/V190I/V190W/V190M/V190Y/V190F/V190L/V190Q/V190S in the heavy chain constant domain CH1; and
    S174F/S174N/S174M/S174A/S174T/S174W/S174R/S174C/S174K/S174H/ S174Y/S174L in the light chain constant domain CL;

At least one mutation, or a combination of at least two mutations, from the following set is introduced into the second Fab region of the antibody or antibody fragment:

    a)S186A/S186T/S186E/S186Q/S186M/S186D/S186K/S186V/S186W/S186 Y in a heavy chain constant domain CH1; and
    T178M/T178Y/T178L/T178I/T178A/T178V/T178S/T178R/T178E in a light chain constant domain CL;

    b) S188Y/S188M/S188T/S188F/S188V/S188L/S188I/S188R in the heavy chain constant domain CH1; and
    S176M/S176L/S176Y/S176A/S176I/S176E/S176T/S176W/S176H/S176V in the light chain constant domain CL;

c) V190T/V190I/V190W/V190M/V190Y/V190F/V190L/V190Q/V190S in the heavy chain constant domain CH1; and

S174F/S174N/S174M/S174A/S174T/S174W/S174R/S174C/S174K/S174H/ S174Y/S174L in the light chain constant domain CL.

In the present invention, unless otherwise specified, the amino acids of the heavy chains and light chains of the antibody are numbered according to Kabat numbering.

It is believed that, without being constrained by theories, in the present invention, the Fab region of the antibody maintains the interaction between side chains through hydrophobic effect, electrostatic forces, and hydrogen bonding via the amino acids at the CH1/CL binding interface. When the above mutations are introduced, they are expected to reduce the occurrence of mispairing between different heavy and light chains, enhance the antibody's binding affinity for its target antigen, increase the manufacturing yield, and result in superior performance characteristics of the antibody.

In some embodiments, the first antigen-binding region (Fab) of the antibody or antibody fragment is modified by the introduction of any one of the following mutations:

8D6:

a) S186T in the heavy chain constant domain CH1 and T178A in the light chain constant domain CL;

b) S188Y in the heavy chain constant domain CH1 and S176E in the light chain constant domain CL;

c) V190F in the heavy chain constant domain CH1 and S174A in the light chain constant domain CL;

or 11B7:

a) S186V in the heavy chain constant domain CH1 and T178L in the light chain constant domain CL;

b) S188L in the heavy chain constant domain CH1 and S176W in the light chain constant domain CL;

c) V190M in the heavy chain constant domain CH1 and S174R in the light chain constant domain CL;

or 11D6:

a) S186W in the heavy chain constant domain CH1 and T178M in the light chain constant domain CL;

b) S188F in the heavy chain constant domain CH1 and S176V in the light chain constant domain CL;

c) V190T in the heavy chain constant domain CH1 and S174K in the light chain constant domain CL;

or 11H2:

a) S186Y in the heavy chain constant domain CH1;

b) S188M in the heavy chain constant domain CH1 and S176V in the light chain constant domain CL;

c) V190F in the heavy chain constant domain CH1.

In some embodiments, the second antigen-binding region (Fab) of the antibody or antibody fragment is modified by introducing any one of the following mutations:

11A10:

a) S186M in the heavy chain constant domain CH1 and T178V in the light chain constant domain CL;

b) S188I in the heavy chain constant domain CH1 and S176H in the light chain constant domain CL;

c) V190W in the heavy chain constant domain CH1 and S174R in the light chain constant domain CL;

or 7A10:

a) S186T in the heavy chain constant domain CH1 and T178V in the light chain constant domain CL;

b) S188R in the heavy chain constant domain CH1;

c) V190F in the heavy chain constant domain CH1;

or 11C12:

a) S186K in the heavy chain constant domain CH1 and T178E in the light chain constant domain CL;

b) S188L in the heavy chain constant domain CH1 and S176M in the light chain constant domain CL;

c) S174Y in the light chain constant domain CL;

or 11A10:

a) S186M in the heavy chain constant domain CH1 and T178V in the light chain constant domain CL;

b) S188I in the heavy chain constant domain CH1 and S176H in the light chain constant domain CL;

c) V190W in the heavy chain constant domain CH1 and S174R in the light chain constant domain CL.

In some embodiments, an A178D mutation is further introduced into the light chain constant domain CL of the first Fab region, and an I188D mutation is further introduced into the heavy chain constant domain CH1 of the second Fab region.

In some embodiments, an L178K mutation is further introduced into the light chain constant domain CL of the first Fab region, and a T192K mutation is further introduced into the heavy chain constant domain CH1 of the second Fab region.

In some embodiments, the mutations introduced into the first Fab region and the second Fab region of the antibody or antibody fragment are the same or different.

Some embodiments of the present invention provide a bispecific antibody, wherein the bispecific antibody comprises an antibody or antibody fragment as described above.

In some embodiments, the light chain constant domain CL of the bispecific antibody is each independently selected from at least one of the following sequences: SEQ ID NO: 3, SEQ ID NO: 7, SEQ ID NO: 11, SEQ ID NO: 15, SEQ ID NO: 19, SEQ ID NO: 23, SEQ ID NO: 27, SEQ ID NO: 31, SEQ ID NO: 35, SEQ ID NO: 39, SEQ ID NO: 43, and SEQ ID NO: 47, SEQ ID NO: 51, SEQ ID NO: 55, SEQ ID NO: 59, SEQ ID NO: 63, SEQ ID NO: 67, SEQ ID NO: 71, SEQ ID NO: 75, SEQ ID NO: 79, SEQ ID NO: 83, SEQ ID NO: 87, SEQ ID NO: 91, SEQ ID NO: 95, SEQ ID NO: 99, SEQ ID NO: 103, SEQ ID NO: 107, SEQ ID NO: 111, or SEQ ID NO: 115.

SEQ ID NO: 3:

TVAAPSVFIFPPSDEQLKSGTASVVCLLNNFYPREAKVQWKVDNALQSGN
SQESVTEQDSKDSTYSLMSMLTLSKADYEKHKVYACEVTHQGLSSPVTKSF
NRGEC;

SEQ ID NO: 7:

TVAAPSVFIFPPSDEQLKSGTASVVCLLNNFYPREAKVQWKVDNALQSGN
SQESVTEQDSKDSTYFLLSMLTLSKADYEKHKVYACEVTHQGLSSPVTKSF
NRGEC;

SEQ ID NO: 11:

TVAAPSVFIFPPSDEQLKSGTASVVCLLNNFYPREAKVQWKVDNALQSGN
SQESVTEQDSKDSTYFLSSYLTLSKADYEKHKVYACEVTHQGLSSPVTKSF
NRGEC;

SEQ ID NO: 15:

TVAAPSVFIFPPSDEQLKSGTASVVCLLNNFYPREAKVQWKVDNALQSGN
SQESVTEQDSKDSTYFLYSLLTLSKADYEKHKVYACEVTHQGLSSPVTKSFN
RGEC;

SEQ ID NO: 19:

TVAAPSVFIFPPSDEQLKSGTASVVCLLNNFYPREAKVQWKVDNALQSGN
SQESVTEQDSKDSTYNLASTLTLSKADYEKHKVYACEVTHQGLSSPVTKSF
NRGEC;

SEQ ID NO: 23:

TVAAPSVFIFPPSDEQLKSGTASVVCLLNNFYPREAKVQWKVDNALQSGN
SQESVTEQDSKDSTYMLISILTLSKADYEKHKVYACEVTHQGLSSPVTKSFN
RGEC;

SEQ ID NO: 27:

TVAAPSVFIFPPSDEQLKSGTASVVCLLNNFYPREAKVQWKVDNALQSGN
SQESVTEQDSKDSTYNLLSALTLSKADYEKHKVYACEVTHQGLSSPVTKSF
NRGEC;

SEQ ID NO: 31:

TVAAPSVFIFPPSDEQLKSGTASVVCLLNNFYPREAKVQWKVDNALQSGN
SQESVTEQDSKDSTYNLYSLLTLSKADYEKHKVYACEVTHQGLSSPVTKSF
NRGEC;

SEQ ID NO: 35:

TVAAPSVFIFPPSDEQLKSGTASVVCLLNNFYPREAKVQWKVDNALQSGN
SQESVTEQDSKDSTYALESALTLSKADYEKHKVYACEVTHQGLSSPVTKSF
NRGEC;

SEQ ID NO: 39:

TVAAPSVFIFPPSDEQLKSGTASVVCLLNNFYPREAKVQWKVDNALQSGN
SQESVTEQDSKDSTYTLASVLTLSKADYEKHKVYACEVTHQGLSSPVTKSF
NRGEC;

SEQ ID NO: 43:

TVAAPSVFIFPPSDEQLKSGTASVVCLLNNFYPREAKVQWKVDNALQSGN
SQESVTEQDSKDSTYWLTSLLTLSKADYEKHKVYACEVTHQGLSSPVTKSF
NRGEC;

SEQ ID NO: 47:

TVAAPSVFIFPPSDEQLKSGTASVVCLLNNFYPREAKVQWKVDNALQSGN
SQESVTEQDSKDSTYSLASILTLSKADYEKHKVYACEVTHQGLSSPVTKSFN
RGEC;

SEQ ID NO: 51:

TVAAPSVFIFPPSDEQLKSGTASVVCLLNNFYPREAKVQWKVDNALQSGN
SQESVTEQDSKDSTYTLYSSLTLSKADYEKHKVYACEVTHQGLSSPVTKSF
NRGEC;

SEQ ID NO: 55:

TVAAPSVFIFPPSDEQLKSGTASVVCLLNNFYPREAKVQWKVDNALQSGN
SQESVTEQDSKDSTYRLWSLLTLSKADYEKHKVYACEVTHQGLSSPVTKSF
NRGEC;

SEQ ID NO: 59:

TVAAPSVFIFPPSDEQLKSGTASVVCLLNNFYPREAKVQWKVDNALQSGN
SQESVTEQDSKDSTYSLTNYLTLSKADYEKHKVYACEVTHQGLSSPVTKSF
NRGEC;

SEQ ID NO: 63:

TVAAPSVFIFPPSDEQLKSGTASVVCLLNNFYPREAKVQWKVDNALQSGN
SQESVTEQDSKDSTYSLYSVLTLSKADYEKHKVYACEVTHQGLSSPVTKSF
NRGEC;

SEQ ID NO: 67:

TVAAPSVFIFPPSDEQLKSGTASVVCLLNNFYPREAKVQWKVDNALQSGN
SQESVTEQDSKDSTYNLSSLLTLSKADYEKHKVYACEVTHQGLSSPVTKSF
NRGEC;

SEQ ID NO: 71:

TVAAPSVFIFPPSDEQLKSGTASVVCLLNNFYPREAKVQWKVDNALQSGN
SQESVTEQDSKDSTYCLTSVLTLSKADYEKHKVYACEVTHQGLSSPVTKSF
NRGEC;

SEQ ID NO: 75:

TVAAPSVFIFPPSDEQLKSGTASVVCLLNNFYPREAKVQWKVDNALQSGN
SQESVTEQDSKDSTYFLSSLLTLSKADYEKHKVYACEVTHQGLSSPVTKSFN
RGEC;

SEQ ID NO: 79:

TVAAPSVFIFPPSDEQLKSGTASVVCLLNNFYPREAKVQWKVDNALQSGN
SQESVTEQDSKDSTYRLHSVLTLSKADYEKHKVYACEVTHQGLSSPVTKSF
NRGEC;

SEQ ID NO: 83:

TVAAPSVFIFPPSDEQLKSGTASVVCLLNNFYPREAKVQWKVDNALQSGN
SQESVTEQDSKDSTYKLVSMLTLSKADYEKHKVYACEVTHQGLSSPVTKSF
NRGEC;

SEQ ID NO: 87:

TVAAPSVFIFPPSDEQLKSGTASVVCLLNNFYPREAKVQWKVDNALQSGN
SQESVTEQDSKDSTYHLVSRLTLSKADYEKHKVYACEVTHQGLSSPVTKSF
NRGEC;

SEQ ID NO: 91:

TVAAPSVFIFPPSDEQLKSGTASVVCLLNNFYPREAKVQWKVDNALQSGN
SQESVTEQDSKDSTYYLMSELTLSKADYEKHKVYACEVTHQGLSSPVTKSF
NRGEC;

SEQ ID NO: 95:

TVAAPSVFIFPPSDEQLKSGTASVVCLLNNFYPREAKVQWKVDNALQSGN
SQESVTEQDSKDSTYNLESMLTLSKADYEKHKVYACEVTHQGLSSPVTKSF
NRGEC;

SEQ ID NO: 99:

TVAAPSVFIFPPSDEQLKSGTASVVCLLNNFYPREAKVQWKVDNALQSGN
SQESVTEQDSKDSTYFLLSSLTLSKADYEKHKVYACEVTHQGLSSPVTKSFN
RGEC;

SEQ ID NO: 103:

TVAAPSVFIFPPSDEQLKSGTASVVCLLNNFYPREAKVQWKVDNALQSGN
SQESVTEQDSKDSTYYLSSILTLSKADYEKHKVYACEVTHQGLSSPVTKSFN
RGEC;

SEQ ID NO: 107:

TVAAPSVFIFPPSDEQLKSGTASVVCLLNNFYPREAKVQWKVDNALQSGN
SQESVTEQDSKDSTYSLVSTLTLSKADYEKHKVYACEVTHQGLSSPVTKSF
NRGEC;

SEQ ID NO: 111:

TVAAPSVFIFPPSDEQLKSGTASVVCLLNNFYPREAKVQWKVDNALQSGN
SQESVTEQDSKDSTYLLWSMLTLSKADYEKHKVYACEVTHQGLSSPVTKSF
NRGEC;

SEQ ID NO: 115:

TVAAPSVFIFPPSDEQLKSGTASVVCLLNNFYPREAKVQWKVDNALQSGN
SQESVTEQDSKDSTYSLSSVLTLSKADYEKHKVYACEVTHQGLSSPVTKSF
NRGEC.

In some embodiments, the heavy chain constant domain CH1 of the bispecific antibody is each independently selected from at least one of the following sequences: SEQ ID NO: 1, SEQ ID NO: 5, SEQ ID NO: 9, SEQ ID NO: 13, SEQ ID NO: 17, SEQ ID NO: 21, SEQ ID NO: 25, SEQ ID NO: 29, SEQ ID NO: 33, SEQ ID NO: 37, SEQ ID NO: 41, SEQ ID NO: 45, SEQ ID NO: 49, SEQ ID NO: 53, SEQ ID NO: 57, SEQ ID NO: 61, SEQ ID NO: 65, SEQ ID NO: 69, SEQ ID NO: 73, SEQ ID NO: 77, SEQ ID NO: 81, SEQ ID NO: 85, SEQ ID NO: 89, SEQ ID NO: 93, SEQ ID NO: 97, SEQ ID NO: 101, SEQ ID NO: 105, SEQ ID NO: 109, or SEQ ID NO: 113.

SEQ ID NO: 1:

ASTKGPSVFPLAPSSKSTSGGTAALGCLVKDYFPEPVTVSWNSGALTSGV
HTFPAVLQSSGLYALYSVVTVPSSSLGTQTYICNVNHKPSNTKVDKKV;

SEQ ID NO: 5:

ASTKGPSVFPLAPSSKSTSGGTAALGCLVKDYFPEPVTVSWNSGALTSGV
HTFPAVLQSSGLYTLMSTVTVPSSSLGTQTYICNVNHKPSNTKVDKKV;

SEQ ID NO: 9:

ASTKGPSVFPLAPSSKSTSGGTAALGCLVKDYFPEPVTVSWNSGALTSGV
HTFPAVLQSSGLYELTSIVTVPSSSLGTQTYICNVNHKPSNTKVDKKV;

SEQ ID NO: 13:

ASTKGPSVFPLAPSSKSTSGGTAALGCLVKDYFPEPVTVSWNSGALTSGV
HTFPAVLQSSGLYQLFSTVTVPSSSLGTQTYICNVNHKPSNTKVDKKV;

SEQ ID NO: 17:

ASTKGPSVFPLAPSSKSTSGGTAALGCLVKDYFPEPVTVSWNSGALTSGV
HTFPAVLQSSGLYMLTSWVTVPSSSLGTQTYICNVNHKPSNTKVDKKV;

SEQ ID NO: 21:

ASTKGPSVFPLAPSSKSTSGGTAALGCLVKDYFPEPVTVSWNSGALTSGV
HTFPAVLQSSGLYSLYSMVTVPSSSLGTQTYICNVNHKPSNTKVDKKV;

SEQ ID NO: 25:

ASTKGPSVFPLAPSSKSTSGGTAALGCLVKDYFPEPVTVSWNSGALTSGV
HTFPAVLQSSGLYDLYSVVTVPSSSLGTQTYICNVNHKPSNTKVDKKV;

SEQ ID NO: 29:

ASTKGPSVFPLAPSSKSTSGGTAALGCLVKDYFPEPVTVSWNSGALTSGV
HTFPAVLQSSGLYKLVSYVTVPSSSLGTQTYICNVNHKPSNTKVDKKV;

SEQ ID NO: 33:

ASTKGPSVFPLAPSSKSTSGGTAALGCLVKDYFPEPVTVSWNSGALTSGV
HTFPAVLQSSGLYTLYSFVTVPSSSLGTQTYICNVNHKPSNTKVDKKV;

SEQ ID NO: 37:

ASTKGPSVFPLAPSSKSTSGGTAALGCLVKDYFPEPVTVSWNSGALTSGV
HTFPAVLQSSGLYMLLSVVTVPSSSLGTQTYICNVNHKPSNTKVDKKV;

SEQ ID NO: 41:

ASTKGPSVFPLAPSSKSTSGGTAALGCLVKDYFPEPVTVSWNSGALTSGV
HTFPAVLQSSGLYTLFSLVTVPSSSLGTQTYICNVNHKPSNTKVDKKV;

SEQ ID NO: 45:

ASTKGPSVFPLAPSSKSTSGGTAALGCLVKDYFPEPVTVSWNSGALTSGV
HTFPAVLQSSGLYVLISQVTVPSSSLGTQTYICNVNHKPSNTKVDKKV;

SEQ ID NO: 49:

ASTKGPSVFPLAPSSKSTSGGTAALGCLVKDYFPEPVTVSWNSGALTSGV
HTFPAVLQSSGLYSLVSYVTVPSSSLGTQTYICNVNHKPSNTKVDKKV;

SEQ ID NO: 53:

ASTKGPSVFPLAPSSKSTSGGTAALGCLVKDYFPEPVTVSWNSGALTSGV
HTFPAVLQSSGLYVLLSMVTVPSSSLGTQTYICNVNHKPSNTKVDKKV;

SEQ ID NO: 57:

ASTKGPSVFPLAPSSKSTSGGTAALGCLVKDYFPEPVTVSWNSGALTSGV
HTFPAVLQSSGLYMLVSTVTVPSSSLGTQTYICNVNHKPSNTKVDKKV;

SEQ ID NO: 61:

ASTKGPSVFPLAPSSKSTSGGTAALGCLVKDYFPEPVTVSWNSGALTSGV
HTFPAVLQSSGLYALFSSVTVPSSSLGTQTYICNVNHKPSNTKVDKKV;

SEQ ID NO: 65:

ASTKGPSVFPLAPSSKSTSGGTAALGCLVKDYFPEPVTVSWNSGALTSGV
HTFPAVLQSSGLYELLSIVTVPSSSLGTQTYICNVNHKPSNTKVDKKV;

SEQ ID NO: 69:

ASTKGPSVFPLAPSSKSTSGGTAALGCLVKDYFPEPVTVSWNSGALTSGV
HTFPAVLQSSGLYTLVSFVTVPSSSLGTQTYICNVNHKPSNTKVDKKV;

SEQ ID NO: 73:

ASTKGPSVFPLAPSSKSTSGGTAALGCLVKDYFPEPVTVSWNSGALTSGV
HTFPAVLQSSGLYSLLSIVTVPSSSLGTQTYICNVNHKPSNTKVDKKV;

SEQ ID NO: 77:

ASTKGPSVFPLAPSSKSTSGGTAALGCLVKDYFPEPVTVSWNSGALTSGV
HTFPAVLQSSGLYMLISWVTVPSSSLGTQTYICNVNHKPSNTKVDKKV;

SEQ ID NO: 81:

ASTKGPSVFPLAPSSKSTSGGTAALGCLVKDYFPEPVTVSWNSGALTSGV
HTFPAVLQSSGLYWLFSTVTVPSSSLGTQTYICNVNHKPSNTKVDKKV;

SEQ ID NO: 85:

ASTKGPSVFPLAPSSKSTSGGTAALGCLVKDYFPEPVTVSWNSGALTSGV
HTFPAVLQSSGLYTLFSSVTVPSSSLGTQTYICNVNHKPSNTKVDKKV;

SEQ ID NO: 89:

ASTKGPSVFPLAPSSKSTSGGTAALGCLVKDYFPEPVTVSWNSGALTSGV
HTFPAVLQSSGLYKLLSVVTVPSSSLGTQTYICNVNHKPSNTKVDKKV;

SEQ ID NO: 93:

ASTKGPSVFPLAPSSKSTSGGTAALGCLVKDYFPEPVTVSWNSGALTSGV
HTFPAVLQSSGLYALISIVTVPSSSLGTQTYICNVNHKPSNTKVDKKV;

SEQ ID NO: 97:

ASTKGPSVFPLAPSSKSTSGGTAALGCLVKDYFPEPVTVSWNSGALTSGV
HTFPAVLQSSGLYSLVSIVTVPSSSLGTQTYICNVNHKPSNTKVDKKV;

SEQ ID NO: 101:

ASTKGPSVFPLAPSSKSTSGGTAALGCLVKDYFPEPVTVSWNSGALTSGV
HTFPAVLQSSGLYTLTSLVTVPSSSLGTQTYICNVNHKPSNTKVDKKV;

SEQ ID NO: 105:

ASTKGPSVFPLAPSSKSTSGGTAALGCLVKDYFPEPVTVSWNSGALTSGV
HTFPAVLQSSGLYYLMSFVTVPSSSLGTQTYICNVNHKPSNTKVDKKV;

SEQ ID NO: 109:

ASTKGPSVFPLAPSSKSTSGGTAALGCLVKDYFPEPVTVSWNSGALTSGV
HTFPAVLQSSGLYTLMSSVTVPSSSLGTQTYICNVNHKPSNTKVDKKV;

SEQ ID NO: 113:

ASTKGPSVFPLAPSSKSTSGGTAALGCLVKDYFPEPVTVSWNSGALTSGV
HTFPAVLQSSGLYTLRSFVTVPSSSLGTQTYICNVNHKPSNTKVDKKV.

In some embodiments, the first Fab region and the second Fab region of the bispecific antibody are each independently selected from at least one of the following sequences:

a light chain constant domain CL of SEQ ID NO: 35 and a heavy chain constant domain CH1 of SEQ ID NO: 33; or

a light chain constant domain CL of SEQ ID NO: 79 and a heavy chain constant domain CH1 of SEQ ID NO: 77; or

a light chain constant domain CL of SEQ ID NO: 55 and a heavy chain constant domain CH1 of SEQ ID NO: 53; or

a light chain constant domain CL of SEQ ID NO: 115 and a heavy chain constant domain CH1 of SEQ ID NO: 113; or

a light chain constant domain CL of SEQ ID NO: 83 and a heavy chain constant domain CH1 of SEQ ID NO: 81; or

a light chain constant domain CL of SEQ ID NO: 91 and a heavy chain constant domain CH1 of SEQ ID NO: 89; or

a light chain constant domain CL of SEQ ID NO: 107 and a heavy chain constant domain CH1 of SEQ ID NO: 105; or

a light chain constant domain CL of SEQ ID NO: 79 and a heavy chain constant domain CH1 of SEQ ID NO: 77.

In some embodiments, the first Fab region and the second Fab region of the bispecific antibody bind to different antigens or two different epitopes of the same antigen.
In some embodiments, the antigen is independently selected from any one or a combination of two of CD2, CD3, CD3E, CD4, CD11, CD11a, CD14, CD16, CD18, CD19, CD20, CD22, CD23, CD25, CD28, CD29, CD30, CD32a, CD32b, CD33 (p67 protein), CD38, CD40, CD40L, CD52, CD54, CD56, CD64, CD80, CD147, GD3, IL-1α, IL-1β, IL-1R, IL-2, IL-2R, IL-4, IL-5, IL-5R, IL-6, IL-6R, IL-8, IL-9, IL-12, IL-13, IL-15, IL-17, IL-17R, IL-18, IL-23, interferon α, interferon β, interferon γ, TNF-α, TNF-β, TNF-R1, TNF-RII, FasL, CD27L, CD30L, 4-1BBL, TRAIL, RANKL, TWEAK, APRIL, BAFF, LIGHT, VEG1, OX40L, TRAIL receptor-1, adenosine receptor, lymphotoxin-β receptor, TACI, BAFF-R, EPO; LFA-3, ICAM-1, ICAM-3, EpCAM, integrin β1, integrin β2, integrin α4/β7, integrin α2, integrin α3, integrin α4, integrin α5, integrin α6, integrin αv, integrin αVβ3, FGFR-3, keratinocyte growth factor, VLA-1, VLA-4, L-selectin, anti-Id, E-selectin, HLA, HLA-DR, CTLA-4, T cell receptor, B7-1, B7-2, VNR integrin, TGFβ1, TGFβ2, eotaxin 1, Blys (B lymphocyte stimulator), complement C5, IgE, factor VII, CD64, CBL, NCA 90, EGFR (ErbB-1), Her1, Her2/neu (ErbB-2), Her3 (ErbB-3), Her4 (ErbB-4), tissue factor, endothelin receptor, VLA-4, hapten NP-cap, NIP-cap, E-selectin, digoxin, placental alkaline phosphatase (PLAP), testicular PLAP-like alkaline phosphatase, transferrin receptor, carcinoembryonic antigen (CEA), CEACAM5, HMFG1, PEM, mucin MUC1, MUC18, heparinase I, human cardiac myosin, tumor-associated glycoprotein-72 (TAG-72), tumor-associated antigen CA125, prostate-specific membrane antigen (PSM-A), high molecular weight melanoma-associated antigen (HMW-MAA), carcinoma-associated antigen, Glycoprotein IIb/IIIa (gpIIb/IIIa), tumor-associated antigen expressing Lewis Y-associated carbohydrate, human cytomegalovirus (HCMV) gH envelope glycoprotein, HIV gp120, HCMV, respiratory syncytial virus RSV F, RSVF Fgp, cytokeratin tumor-associated antigen, Hep B gp120, CMV, gpIIb/IIIa, HIV IIIB gp120V3 loop, respiratory syncytial virus (RSV) Fgp, herpes simplex virus (HSV) gD glycoprotein, HSV gB glycoprotein, HCMV gB envelope glycoprotein, *Clostridium perfringens* toxin, CD133, CD138, OX40, GITR, PD-1, PDL1, PD-L2, CTLA-4, KIR, LAG-3, TCRα, TCRβ, TCRγ, TCRδ, VEGF, EGF, VEGFR, EGFR, EpCAM, mesothelin, glypicans, Erb1, Erb2, B7-H3, ICOS, BMP1, BMP2, BMP3B, BMP4, CSF1, GM-CSF, FGF1, FGF2, FGF3, FGF4, PDGFR, TIGIT, CS1, TWEAK, CCL1, CCL2, CCL3, CCL13, CXCL1, CXCL2, CXCL3, IP-10, fucosyl-GM1, IGF1, IGF2, IGF1R, IGF2R, RANK ligand, DLL-4, GM-CSFR, ADAMS, myostatin, PCSK9, CXCR4, MIF, or PEG2.

In some embodiments, the first Fab region of the bispecific antibody binds to any one of CD3, CD3E, CD16, CD28, CD47, CD27, CD40, OX40, GITR, ICOS, 4-1BB, PD-1, PD-L1, GITR, TIGIT, TIM3, LAG3, B7H3, CTLA4, VISTA, BTLA, HVEM, NKG2A, or NECL.

In some embodiments, the second Fab region of the bispecific antibody binds to any one of CD2, CD4, CD11, CD11a, CD14, CD18, CD19, CD20, CD22, CD23, CD25, CD29, CD30, CD32a, CD32b, CD33, CD38, CD52, CD54, CD56, CD64, CD80, CD147, GD3, IL-1α, IL-1β, IL-1R, IL-2, IL-2R, IL-4, IL-5, IL-5R, IL-6, IL-6R, IL-8, IL-9, IL-12, IL-13, IL-15, IL-17, IL-17R, IL-18, IL-23, interferon α, interferon β, interferon γ, TNF-α, TNF-β, TNF-R1, TNF-RII, TACI, BAFF-R, EPO; LFA-3, GPC-3, ICAM-1, ICAM-3, complement C5, IgE, factor VII, CD64, CBL, NCA 90, Her1, Her2, Her3, Her4, carcinoembryonic antigen (CEA), tumor-associated glycoprotein-72 (TAG-72), tumor-associated antigen CA125, prostate-specific membrane antigen (PSM-A), high molecular weight melanoma-associated antigen (HMW-MAA), carcinoma-associated antigen, or tumor-associated antigen expressing Lewis Y-associated carbohydrate.

In some embodiments, the antigen to which the first Fab region of the bispecific antibody binds is CD3, and the antigen to which the second Fab region binds is GPC3.

In some embodiments, the bispecific antibody comprises a first heavy chain and a first light chain binding to CD3, and a second heavy chain and a second light chain binding to GPC3.

In some embodiments, the bispecific antibody is selected from any one of the following amino acid sequences:

the first heavy chain has an amino acid sequence set forth in SEQ ID NO: 129, and the first light chain has an amino acid sequence set forth in SEQ ID NO: 127; the second heavy chain has an amino acid sequence set forth in SEQ ID NO: 133, and the second light chain has an amino acid sequence set forth in SEQ ID NO: 131; or

the first heavy chain has an amino acid sequence set forth in SEQ ID NO: 145, and the first light chain has an amino acid sequence set forth in SEQ ID NO: 143; the second heavy chain has an amino acid sequence set forth in SEQ ID NO: 149, and the second light chain has an amino acid sequence set forth in SEQ ID NO: 147; or

the first heavy chain has an amino acid sequence set forth in SEQ ID NO: 137, and the first light chain has an amino acid sequence set forth in SEQ ID NO: 135; the second heavy chain has an amino acid sequence set forth in SEQ ID NO: 141, and the second light chain has an amino acid sequence set forth in SEQ ID NO: 139; or

the first heavy chain has an amino acid sequence set forth in SEQ ID NO: 153, and the first light chain has an amino acid sequence set forth in SEQ ID NO: 151; the second heavy chain has an amino acid sequence set forth in SEQ ID NO: 157, and the second light chain has an amino acid sequence set forth in SEQ ID NO: 155.

SEQ ID NO: 129:

EVQLVESGGGLVQPGGSLKLSCAASGFTFNTYAMNWVRQAPGKGLEWV
ARIRSKYNNYATYYADSVKDRFTISRDDSKNTAYLQMNNLKTEDTAMYYCV
RHGNFGHSYVSWFAYWGQGTLVTVSSASTKGPSVFPLAPSSKSTSGGTAA
LGCLVKDYFPEPVTVSWNSGALTSGVHTFPAVLQSSGLYTLYSFVTVPSSSL
GTQTYICNVNHKPSNTKVDKKVEPKSCDKTHTCPPCPAPEAAGGPSVFLFP
PKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTKKKL
DSDGSFFLYSKLTVDKSRWQQGNVFSCSVMHEALHNHYTQKSLSLSPGK;

SEQ ID NO: 127:

DIQMTQEPSLTTSPGGTVTLTCRSSTGAVTTSNYANWVQEKPGQAPRGLI
GGTNKRAPGTPARFSGSLIGGKAALTITGVQPEDEAIYFCALWYSNLWVFG
GGTKLEIKRTVAAPSVFIFPPSDEQLKSGTASVVCLLNNFYPREAKVQWKVD

NALQSGNSQESVTEQDSKDSTYALESALTLSKADYEKHKVYACEVTHQGLS
SPVTKSFNRGEC;

SEQ ID NO: 133:

QVQLVQSGAEVKKPGASVKLSCKASGYTFTDYEMHWVKQTPGKGLKWI
GALDPKTGDTAYAQKFQGRATLTADTSTDTAYMELSSLRSEDSAVYYCTRF
YSYTYWGQGTLVTVSSASTKGPSVFPLAPSSKSTSGGTAALGCLVKDYFPE
PVTVSWNSGALTSGVHTFPAVLQSSGLYMLISWVTVPSSSLGTQTYICNVN
HKPSNTKVDKKVEPKSCDKTHTCPPCPAPEAAGGPSVFLFPPKPKDTLMIS
RTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRE
EMTKNQVSLTCLVKGFYPSDIAVEWESNGQPENNYKTDDDVLDSDGSFFLY
SKLTVDKSRWQQGNVFSCSVMHEALHNHYTQKSLSLSPGK;

SEQ ID NO: 131:

DIVLTQSPLSLTVTPGEPASISCRSSESLVHSNGNTYLSWLQQRPGQPPR
LLIYKISNRFSGVPDRFSGSGAGTDFTLKISRVEAEDVGVYYCVQVSSFPYTF
GQGTKVEIKTVAAPSVFIFPPSDEQLKSGTASVVCLLNNFYPREAKVQWKVD
NALQSGNSQESVTEQDSKDSTYRLHSVLTLSKADYEKHKVYACEVTHQGLS
SPVTKSFNRGEC;

SEQ ID NO: 145:

EVQLVESGGGLVQPGGSLKLSCAASGFTFNTYAMNWVRQAPGKGLEWV
ARIRSKYNNYATYYADSVKDRFTISRDDSKNTAYLQMNNLKTEDTAMYYCV
RHGNFGHSYVSWFAYWGQGTLVTVSSASTKGPSVFPLAPSSKSTSGGTAA
LGCLVKDYFPEPVTVSWNSGALTSGVHTFPAVLQSSGLYTLYSFVTVPSSSL
GTQTYICNVNHKPSNTKVDKKVEPKSCDKTHTCPPCPAPEAAGGPSVFLFP
PKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTKKKL
DSDGSFFLYSKLTVDKSRWQQGNVFSCSVMHEALHNHYTQKSLSLSPGK;

SEQ ID NO: 143:


DIQMTQEPSLTTSPGGTVTLTCRSSTGAVTTSNYANWVQEKPGQAPRGLI
GGTNKRAPGTPARFSGSLIGGKAALTITGVQPEDEAIYFCALWYSNLWVFG
GGTKLEIKRTVAAPSVFIFPPSDEQLKSGTASVVCLLNNFYPREAKVQWKVD
NALQSGNSQESVTEQDSKDSTYALESDLTLSKADYEKHKVYACEVTHQGLS
SPVTKSFNRGEC;

SEQ ID NO: 149:


QVQLVQSGAEVKKPGASVKLSCKASGYTFTDYEMHWVKQTPGKGLKWI
GALDPKTGDTAYAQKFQGRATLTADTSTDTAYMELSSLRSEDSAVYYCTRF
YSYTYWGQGTLVTVSSASTKGPSVFPLAPSSKSTSGGTAALGCLVKDYFPE
PVTVSWNSGALTSGVHTFPAVLQSSGLYMLDSWVTVPSSSLGTQTYICNVN
HKPSNTKVDKKVEPKSCDKTHTCPPCPAPEAAGGPSVFLFPPKPKDTLMIS
RTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRE

EMTKNQVSLTCLVKGFYPSDIAVEWESNGQPENNYKTDDDVLDSDGSFFLY
SKLTVDKSRWQQGNVFSCSVMHEALHNHYTQKSLSLSPGK;

SEQ ID NO: 147:


DIVLTQSPLSLTVTPGEPASISCRSSESLVHSNGNTYLSWLQQRPGQPPR
LLIYKISNRFSGVPDRFSGSGAGTDFTLKISRVEAEDVGVYYCVQVSSFPYTF
GQGTKVEIKTVAAPSVFIFPPSDEQLKSGTASVVCLLNNFYPREAKVQWKVD
NALQSGNSQESVTEQDSKDSTYRLHSVLTLSKADYEKHKVYACEVTHQGLS
SPVTKSFNRGEC;

SEQ ID NO: 137:

EVQLVESGGGLVQPGGSLKLSCAASGFTFNTYAMNWVRQAPGKGLEWV
ARIRSKYNNYATYYADSVKDRFTISRDDSKNTAYLQMNNLKTEDTAMYYCV
RHGNFGHSYVSWFAYWGQGTLVTVSSASTKGPSVFPLAPSSKSTSGGTAA
LGCLVKDYFPEPVTVSWNSGALTSGVHTFPAVLQSSGLYVLLSMVTVPSSS
LGTQTYICNVNHKPSNTKVDKKVEPKSCDKTHTCPPCPAPEAAGGPSVFLF
PPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREP
QVYTLPPSREEMTKNQVSLTCLVKGFYPSDIAVEWESNGQPENNYKTTKKK
LDSDGSFFLYSKLTVDKSRWQQGNVFSCSVMHEALHNHYTQKSLSLSPGK;

SEQ ID NO: 135:


DIQMTQEPSLTTSPGGTVTLTCRSSTGAVTTSNYANWVQEKPGQAPRGLI
GGTNKRAPGTPARFSGSLIGGKAALTITGVQPEDEAIYFCALWYSNLWVFG
GGTKLEIKRTVAAPSVFIFPPSDEQLKSGTASVVCLLNNFYPREAKVQWKVD
NALQSGNSQESVTEQDSKDSTYRLWSLLTLSKADYEKHKVYACEVTHQGLS
SPVTKSFNRGEC;

SEQ ID NO: 141:


QVQLVQSGAEVKKPGASVKLSCKASGYTFTDYEMHWVKQTPGKGLKWI
GALDPKTGDTAYAQKFQGRATLTADTSTDTAYMELSSLRSEDSAVYYCTRF
YSYTYWGQGTLVTVSSASTKGPSVFPLAPSSKSTSGGTAALGCLVKDYFPE
PVTVSWNSGALTSGVHTFPAVLQSSGLYTLRSFVTVPSSSLGTQTYICNVNH
KPSNTKVDKKVEPKSCDKTHTCPPCPAPEAAGGPSVFLFPPKPKDTLMISRT
PEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSREE
MTKNQVSLTCLVKGFYPSDIAVEWESNGQPENNYKTDDDVLDSDGSFFLYS
KLTVDKSRWQQGNVFSCSVMHEALHNHYTQKSLSLSPGK;

SEQ ID NO: 139:


DIVLTQSPLSLTVTPGEPASISCRSSESLVHSNGNTYLSWLQQRPGQPPR
LLIYKISNRFSGVPDRFSGSGAGTDFTLKISRVEAEDVGVYYCVQVSSFPYTF
GQGTKVEIKTVAAPSVFIFPPSDEQLKSGTASVVCLLNNFYPREAKVQWKVD
NALQSGNSQESVTEQDSKDSTYSLSSTLTLSKADYEKHKVYACEVTHQGLS
SPVTKSFNRGEC;

SEQ ID NO: 153:

EVQLVESGGGLVQPGGSLKLSCAASGFTFNTYAMNWVRQAPGKGLEWV
ARIRSKYNNYATYYADSVKDRFTISRDDSKNTAYLQMNNLKTEDTAMYYCV
RHGNFGHSYVSWFAYWGQGTLVTVSSASTKGPSVFPLAPSSKSTSGGTAA
LGCLVKDYFPEPVTVSWNSGALTSGVHTFPAVLQSSGLYVLLSMVTVPSSS
LGTQTYICNVNHKPSNTKVDKKVEPKSCDKTHTCPPCPAPEAAGGPSVFLF
PPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREP
QVYTLPPSREEMTKNQVSLTCLVKGFYPSDIAVEWESNGQPENNYKTTKKK
LDSDGSFFLYSKLTVDKSRWQQGNVFSCSVMHEALHNHYTQKSLSLSPGK;

SEQ ID NO: 151:


DIQMTQEPSLTTSPGGTVTLTCRSSTGAVTTSNYANWVQEKPGQAPRGLI
GGTNKRAPGTPARFSGSLIGGKAALTITGVQPEDEAIYFCALWYSNLWVFG
GGTKLEIKRTVAAPSVFIFPPSDEQLKSGTASVVCLLNNFYPREAKVQWKVD
NALQSGNSQESVTEQDSKDSTYRLWSKLTLSKADYEKHKVYACEVTHQGL
SSPVTKSFNRGEC;


SEQ ID NO: 157:


QVQLVQSGAEVKKPGASVKLSCKASGYTFTDYEMHWVKQTPGKGLKWI
GALDPKTGDTAYAQKFQGRATLTADTSTDTAYMELSSLRSEDSAVYYCTRF
YSYTYWGQGTLVTVSSASTKGPSVFPLAPSSKSTSGGTAALGCLVKDYFPE
PVTVSWNSGALTSGVHTFPAVLQSSGLYTLRSFVKVPSSSLGTQTYICNVNH
KPSNTKVDKKVEPKSCDKTHTCPPCPAPEAAGGPSVFLFPPKPKDTLMISRT
PEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSREE
MTKNQVSLTCLVKGFYPSDIAVEWESNGQPENNYKTDDDVLDSDGSFFLYS
KLTVDKSRWQQGNVFSCSVMHEALHNHYTQKSLSLSPGK;


SEQ ID NO: 155:


DIVLTQSPLSLTVTPGEPASISCRSSESLVHSNGNTYLSWLQQRPGQPPR
LLIYKISNRFSGVPDRFSGSGAGTDFTLKISRVEAEDVGVYYCVQVSSFPYTF
GQGTKVEIKTVAAPSVFIFPPSDEQLKSGTASVVCLLNNFYPREAKVQWKVD
NALQSGNSQESVTEQDSKDSTYSLSSTLTLSKADYEKHKVYACEVTHQGLS
SPVTKSFNRGEC.

In some embodiments, the bispecific antibody is derived from IgG, IgA, IgM, IgE, or IgD.

In some embodiments, the bispecific antibody is derived from IgG1, IgG2, IgG3, or IgG4.

Some embodiments of the present invention further provide a nucleic acid molecule, the nucleic acid molecule encoding an antibody or antibody fragment, and/or a bispecific antibody, as described in any of the preceding embodiments.

In some embodiments, the present invention further includes nucleic acid sequences encoding these polypeptide chains. During expression of antibodies, nucleic acid sequences are inserted into suitable vectors, the vectors including but not limited to: plasmids, phage expression vectors, Cosmid plasmids, artificial chromosomes, bacteriophages, animal viruses, and others. The expression vectors contain elements for regulating expression, including but not limited to promoters, transcription start sequences, enhancers, signal peptide sequences, and others. The promoters include but are not limited to, T7 promoter, T3 promoter, SP6 promoter, β-actin promoter, EF-1α promoter, CMV promoter, and SV40 promoter. The expression vectors may be transferred into host cells using appropriate methods known in the art, including but not limited to calcium phosphate precipitation, polyethylenimine transfection, lipid transfection, electroporation, and PEI (polyethylenimine) transfection.

Some embodiments of the present invention further provide an expression vector, wherein the expression vector comprises a nucleic acid molecule as described above.

Some embodiments of the present invention further provide a recombinant cell, wherein the recombinant cell expresses an antibody or antibody fragment, and/or a bispecific antibody, as described in any of the preceding embodiments.

In some embodiments, the nucleic acid molecule described above is integrated into the genome of the recombinant cell.

In some embodiments, the recombinant cell comprises an expression vector as described above.

Some embodiments of the present invention further provide a method for preparing an antibody or antibody fragment as described in any of the preceding embodiments, comprising the steps:

inserting a nucleic acid molecule encoding the antibody or antibody fragment into a plasmid, transferring the plasmid into competent cells, and subsequently selecting monoclonal cells for screening;

purifying the expression vector from the screened positive clone, transferring the vector into recipient cells, and obtaining recombinant cells through screening;

culturing the recombinant cells, collecting the culture supernatant, and isolating and purifying the antibody or antibody fragment from the collected supernatant.

Some embodiments of the present invention further provide an antibody conjugate, which comprises an antibody or antibody fragment, and/or a bispecific antibody, as described in any of the preceding embodiments, and a conjugated marker.

In some embodiments, the conjugated marker comprises any one or a combination of at least two of a cytotoxin, a radioisotope, a fluorescent marker, a luminescent substance, a chromogenic substance, or an enzyme.

Some embodiments of the present invention further provide a pharmaceutical composition, which comprises any one or a combination of at least two of an antibody or antibody fragment, and/or a bispecific antibody, as described in any of the preceding embodiments, or the antibody conjugate.

The present invention relates to a pharmaceutical composition comprising an antibody or antibody fragment, a bispecific antibody or an antibody conjugate of the present invention, and optionally a pharmaceutically acceptable carrier, a surfactant, and/or a diluent. In some embodiments, in addition to the antibody, the bispecific antibody, or the antibody conjugate of the present invention, the pharmaceutic composition comprises one or more additional therapeutic agents. In some embodiments, the additional therapeutic agents include but are not limited to, chemotherapeutic agents, growth inhibitors, cytotoxic agents, reagents for radiation therapy, anti-angiogenic agents, apoptotic agents, anti-tubulin agents, and other agents for treating cancer.

In some embodiments, the pharmaceutical composition further comprises any one or more, or a combination of at least two of a pharmaceutically acceptable carrier, a surfactant, a diluent, or an excipient, either individually or in combination.

Some embodiments of the present invention further provide the use of any one or more, or a combination of at least two of the antibody or antibody fragment, and/or a bispecific antibody, as described in any of the preceding embodiments, or the pharmaceutical composition described above in the preparation of a medicament for the treatment of a disease.

In some embodiments, the disease includes cancer.

Some embodiments of the present invention further provide the use of any one or a combination of at least two of the antibody or antibody fragment described above, the bispecific antibody described above, the antibody conjugate, or the pharmaceutic composition, for the treatment of a disease.

In some embodiments, the disease includes cancer.

Some embodiments of the present invention further provide a method for treating a disease, the method comprising: administering any one or a combination of at least two of the antibody or antibody fragment, and/or a bispecific antibody, as described in any of the preceding embodiments, the antibody conjugate, or the pharmaceutical composition to a subject in need thereof.

In some embodiments, the disease includes cancer.

The present invention provides a bispecific antibody and use thereof, in which mispairing between light and heavy chains is reduced by mutating amino acids at the interface between the heavy chain CH1 and the light chain CL in terms of amino acid size and charge. This modification improves the antibody's ability to bind to a specific antigen and increases the yield of the correct product, thereby offering high application value.

(1) In the present invention, the probability of correct pairing of heavy/light chains in heterodimeric bispecific antibodies targeting specific antigens is increased by mutating amino acid sites at the CH1/CL binding interfaces. This mutation strategy not only increases the binding affinity and specificity for the target antigens but also improves the yield of the desired target bispecific antibodies.

(2) The anti-GPC3×CD3 tetra-chain bispecific antibodies MutA, MutB, MutA+D-D, and MutB+K-K constructed in the present invention, exhibit well-paired light chains with their corresponding heavy chains, whereby target bispecific antibody molecules can be effectively produced, which have a better binding activity to targeted antigens, increased T cell activation potential, superior tumor cell-killing effects, and thus have a high application value.

## EXAMPLES

### Example 1. Evaluation of Modifiable Amino Acid Sites at CH1/CL Binding Interface

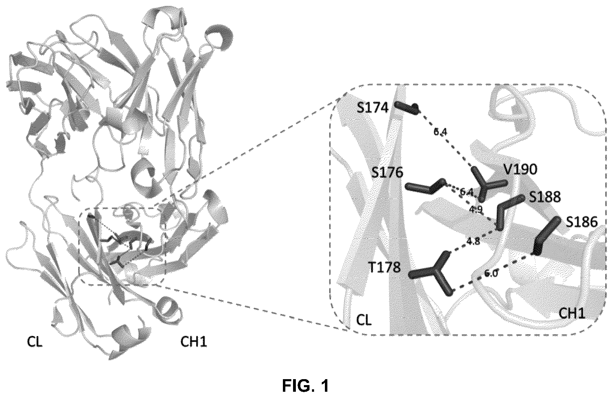

The amino acids at the CH1/CL binding interface of antibody's Fab fragment maintain the interaction between side chains through various non-covalent interactions, such as hydrophobic interaction, charge interaction, and hydrogen bonding. These interactions are typically enabled by the proximity of amino acids, within a range of about 4 to 6 angstroms (Å). In this example, potentially modifiable amino acid residues in each CH1 and CL domain of a bispecific antibody were obtained by analyzing the protein crystal structure at the CH1/CL binding interface. The crystal structure of the Fab fragment (PDB ID: 3EO9) was analyzed by PyMOL software, revealing the binding interface of the E-strand within the CH1-CL domain as depicted in Figure 1. The amino acid residues with strong interactions are summarized in Table 1 (Summary of Modifiable Sites).

In this invention, unless otherwise specified, amino acid numbers were recorded according to Kabat numbering. For the purposes of this example, the heavy (H) chain is based on the IgG1 isotype, while the light (L) chain can be of the IgK (Kappa, κ) or Lambda (λ) type.

Table 1

| Modifiable Amino Acid Residues at CH1-CL(Kappa) E-Strand binding Interface | | | |
|---|---|---|---|
| Position in CH1 | | Position in CL | |
| Amino Acid Name | Kabat Number | Amino Acid Name | Kabat Number |
| S | 186 | T | 178 |
| S | 188 | S | 176 |
| V | 190 | S | 174 |

### Example 2. Screening of Mutations of Amino Acid Residues at CH1/CL Binding Interface

To identify specific mutations at the CH1/CL interfaces that facilitate heavy-light chain pairing, the following methods were employed to screen for mutation combinations with effective pairing at the CH1/CL interfaces.

Using the methods described by Hust et al. (Methods in Molecular Biology-Phage Display Volume 1701 II Construction of Synthetic Antibody Phage-Display Libraries[J]. 2018.), primers for random mutation of amino acids were designed to construct a phage display library of Fab. This library features random mutations at the modifiable amino acid residues on the CH1-kCL binding interface, as listed in Table 1.

After three rounds of screening with biotinylated antigens, involving steps of release, packaging, and purification of the phage display library, the selected clones were evaluated using an enzyme-linked immunosorbent assay (ELISA). The binding affinity of the mutant clones to the antigens was determined by measuring the absorbance values at a wavelength of 450 nm (OD450). The combinations of mutations with strong interaction in the display library exhibited enhanced antigen-binding abilities. Using a wild-type CH1-kCL clone as a reference, we identified a series of monoclonal variants with mutated amino acids at the CH1-kCL interfaces, ultimately showing improved antigen-binding activity. These clones, featuring mutated amino acids at the CH1-kCL interfaces that enhance antigen-binding activity, are detailed in Table 2. Table 2 shows the $OD_{450}$ and $\Delta OD_{450}$ values of the wild-type and corresponding mutated clones, where $OD_{450}$ represents the absorbance value of a phage clone bound to the antigen, and $\Delta OD_{450}$ represents the difference between the $OD_{450}$ of the mutated clone and that of the wild-type reference.

The combinations of amino acid residue mutations at the CH1/CL-binding interfaces of the clones listed in Table 2 were summarized in Table 3 (Summary of Mutations of Amino Acid Residues). Such mutations could enhance the correct pairing of heavy/light chains against specific antigens in heterodimeric bispecific antibodies.

Table 2

| Clones with Amino Acid Residues at CH1-kCL binding interfaces with Enhanced Antigen-binding Activity | | |
|---|---|---|
| Clone Number | $OD_{450}$ | $\Delta OD_{450}$ |
| Wild-type CH1-KCL | 0.382 | 0 |
| 7D5 | 1.108 | 0.726 |
| 7D10 | 1.155 | 0.773 |
| 7D11 | 0.610 | 0.228 |
| 7F4 | 1.236 | 0.854 |
| 7F10 | 1.199 | 0.817 |
| 7G1 | 1.203 | 0.821 |
| 7H11 | 0.997 | 0.615 |
| 8C10 | 0.746 | 0.364 |
| 8D6 | 0.519 | 0.137 |
| 8E10 | 1.078 | 0.696 |
| 8F11 | 0.883 | 0.501 |
| 8G5 | 0.943 | 0.561 |
| 8H5 | 0.822 | 0.44 |
| 11B7 | 0.714 | 0.332 |
| 11C8 | 1.173 | 0.791 |
| 11F5 | 0.618 | 0.236 |
| 11H7 | 0.745 | 0.363 |
| 12B2 | 0.848 | 0.466 |
| 12D6 | 0.610 | 0.228 |
| 11A10 | 1.131 | 0.749 |
| 11D6 | 1.074 | 0.692 |
| 11C3 | 1.016 | 0.634 |
| 11C12 | 1.039 | 0.657 |
| 11F6 | 1.047 | 0.665 |
| 11F8 | 1.169 | 0.787 |
| 11H5 | 0.786 | 0.404 |
| 11H2 | 0.917 | 0.535 |
| 12C3 | 0.786 | 0.404 |
| 7A10 | 1.124 | 0.742 |

Table 3

| Combinations of Amino Acid Residue Mutations at CH1-CL(Kappa) Binding Interface | | | | |
|---|---|---|---|---|
| Colon Number | Mutation in CH1 | Number | Mutation in CL | Number |
| 7D5 | S186A, S188Y | SEQ ID NO. 1 | S176M, T178M | SEQ ID NO. 3 |

(continued)

| Combinations of Amino Acid Residue Mutations at CH1-CL(Kappa) Binding Interface | | | | |
|---|---|---|---|---|
| Colon Number | Mutation in CH1 | Number | Mutation in CL | Number |
| 7D10 | S186T, S188M, V190T | SEQ ID NO. 5 | S174F, S176L, T178M | SEQ ID NO. 7 |
| 7D11 | S186E, S188T, V190I | SEQ ID NO. 9 | S174F, T178Y | SEQ ID NO. 11 |
| 7F4 | S186Q, S188F, V190T | SEQ ID NO. 13 | S174F, S176Y, T178L | SEQ ID NO. 15 |
| 7F10 | S186M, S188T, V190W | SEQ ID NO. 17 | S174N, S176A | SEQ ID NO. 19 |
| 7G1 | S188Y, V190M | SEQ ID NO. 21 | S174M, S176I, T178I | SEQ ID NO. 23 |
| 7H11 | S186D, S188Y | SEQ ID NO. 25 | S174N, S176L, T178A | SEQ ID NO. 27 |
| 8C10 | S186K, S188V, V190Y | SEQ ID NO. 29 | S174N, S176Y, T178L | SEQ ID NO. 31 |
| 8D6 | S186T, S188Y, V190F | SEQ ID NO. 33 | S174A, S176E, T178A | SEQ ID NO. 35 |
| 8E10 | S186M, S188L | SEQ ID NO. 37 | S174T, S176A, T178V | SEQ ID NO. 39 |
| 8F11 | S186T, S188F, V190L | SEQ ID NO. 41 | S174W, S176T, T178L | SEQ ID NO. 43 |
| 8G5 | S186V, S188I, V190Q | SEQ ID NO. 45 | S176A, T178I | SEQ ID NO. 47 |
| 8H5 | S188V, V190Y | SEQ ID NO. 49 | S174T, S176Y, T178S | SEQ ID NO. 51 |
| 11B7 | S186V, S188L, V190M | SEQ ID NO. 53 | S174R, S176W, T178L | SEQ ID NO. 55 |
| 11C8 | S186M, S188V, V190T | SEQ ID NO. 57 | S176T, T178Y | SEQ ID NO. 59 |
| 11F5 | S186A, S188F, V190S | SEQ ID NO. 61 | S176Y, T178V | SEQ ID NO. 63 |
| 11H7 | S186E, S188L, V190I | SEQ ID NO. 65 | S174N, T178L | SEQ ID NO. 67 |
| 12B2 | S186T, S188V, V190F | SEQ ID NO. 69 | S174C, S176Y, T178V | SEQ ID NO. 71 |
| 12D6 | S188L, V190I | SEQ ID NO. 73 | S174F, T178L | SEQ ID NO. 75 |
| 11A10 | S186M, S188I, V190W | SEQ ID NO. 77 | S174R, S176H, T178V | SEQ ID NO. 79 |
| 11D6 | S186W, S188F, V190T | SEQ ID NO. 81 | S174K, S176V, T178M | SEQ ID NO. 83 |
| 11C3 | S186T, S188F, V190S | SEQ ID NO. 85 | S174H, S176V, T178R | SEQ ID NO. 87 |
| 11C12 | S186K, S188L | SEQ ID NO. 89 | S174Y, S176M, T178E | SEQ ID NO. 91 |
| 11F6 | S186A, S188I, V190I | SEQ ID NO. 93 | S174N, S176E, T178M | SEQ ID NO. 95 |
| 11F8 | S188V, V190I | SEQ ID NO. 97 | S174F, S176L, T178S | SEQ ID NO. 99 |
| 11H5 | S186T, S188T, V190L | SEQ ID NO. 101 | S174Y, T178I | SEQ ID NO. 103 |
| 11H2 | S186Y, S188M, V190F | SEQ ID NO. 105 | S176V | SEQ ID NO. 107 |
| 12C3 | S186T, S188M, V190S | SEQ ID NO. 109 | S174L, S176W, T178M | SEQ ID NO. 111 |
| 7A10 | S186T, S188R, V190F | SEQ ID NO. 113 | T178V | SEQ ID NO. 115 |

## Example 3. Design of Tetra-Chain Bispecific Antibody and Construction of Expression Vector

(1) Design of Tetra-Chain Bispecific Antibody

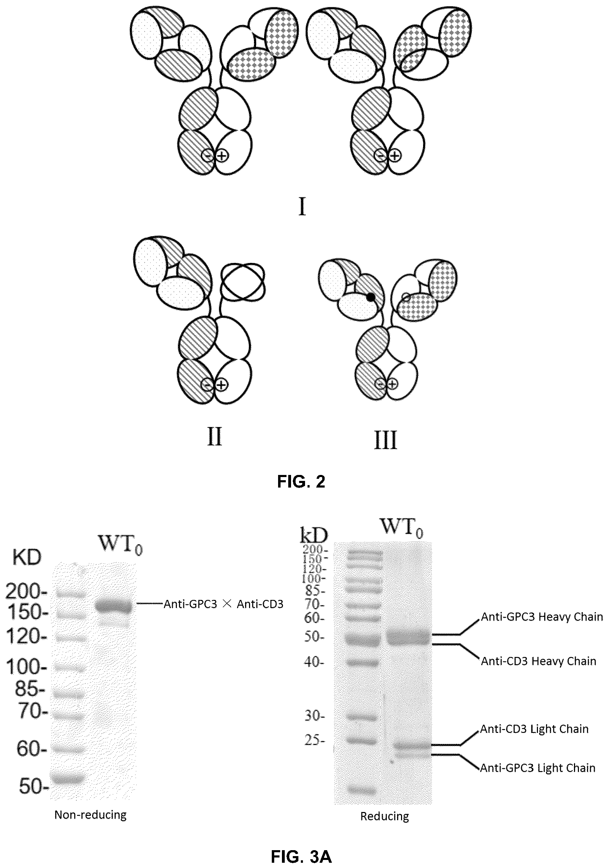

The bispecific antibody designed was a "Y"-shaped IgG antibody (see FIG. 2 for its schematic structural diagram), comprising two heterologous heavy chains and two heterologous light chains, which formed two Fabs and a Fc domain of the antibody.

In this example, the selected target antigens included GPC3 and CD3 but were not limited to this combination. The first Fab region of the bispecific antibody binds to human CD3, while the second Fab region binds to human GPC3. The specific binding to human CD3 included a first heavy chain (SEQ ID NO: 119) and a first light chain (SEQ ID NO: 117), while the specific binding to human GPC3 included a second heavy chain (SEQ ID NO: 123) and a second light chain (SEQ ID NO: 121). The Fc domain of the antibody was modified following the method described in Patent No.

CN102471378B, where mutations of P395K, P396K, and V397K were introduced into the heavy chain for binding to human CD3 (labeled as OA), while mutations of T394D, P395D, and P396D were introduced into the heavy chain for binding to human GPC3 (labeled as OB). This resulted in the formation of a heterodimer composed of two different heavy chains. This bispecific antibody, referred to as negative control $WT_0$, consists of two heterologous light chains randomly paired with the heavy chains. Its structural diagram can be found in III of FIG. 2.

Amino Acid Sequence (SEQ ID NO: 117) of Anti-CD3 Light Chain:

DIQMTQEPSLTTSPGGTVTLTCRSSTGAVTTSNYANWVQEKPGQAPRGLI
GGTNKRAPGTPARFSGSLIGGKAALTITGVQPEDEAIYFCALWYSNLWVFG
GGTKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLS
SPVTKSFNRGEC;

Amino Acid Sequence (SEQ ID NO: 119) of Anti-CD3 Heavy Chain:

EVQLVESGGGLVQPGGSLKLSCAASGFTFNTYAMNWVRQAPGKGLEWV
ARIRSKYNNYATYYADSVKDRFTISRDDSKNTAYLQMNNLKTEDTAMYYCV
RHGNFGTSYVSWFAYWGQGTLVTVSSASTKGPSVFPLAPSSKSTSGGTAA
LGCLVKDYFPEPVTVSWNSGALTSGVHTFPAVLQSSGLYSLSSVVTVPSSSL
GTQTYICNVNHKPSNTKVDKKVEPKSCDKTHTCPPCPAPEAAGGPSVFLFP
PKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTKKKL
DSDGSFFLYSKLTVDKSRWQQGNVFSCSVMHEALHNHYTQKSLSLSPGK;

Amino Acid Sequence (SEQ ID NO: 121) of Anti-GPC3 Light Chain:

DIVLTQSPLSLTVTPGEPASISCRSSESLVHSNGNTYLSWLQQRPGQPPR
LLIYKISNRFSGVPDRFSGSGAGTDFTLKISRVEAEDVGVYYCVQVSSFPYTF
GQGTKVEIKTVAAPSVFIFPPSDEQLKSGTASVVCLLNNFYPREAKVQWKVD
NALQSGNSQESVTEQDSKDSTYSLSSTLTLSKADYEKHKVYACEVTHQGLS
SPVTKSFNRGEC;

Amino Acid Sequence (SEQ ID NO: 123) of Anti-GPC3 Heavy Chain:

QVQLVQSGAEVKKPGASVKLSCKASGYTFTDYEMHWVKQTPGKGLKWI
GALDPKTGDTAYAQKFQGRATLTADTSTDTAYMELSSLRSEDSAVYYCTRF
YSYTYWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNH

KPSNTKVDKKVEPKSCDKTHTCPPCPAPEAAGGPSVFLFPPKPKDTLMISRT
PEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSREE
MTKNQVSLTCLVKGFYPSDIAVEWESNGQPENNYKTDDDVLDSDGSFFLYS
KLTVDKSRWQQGNVFSCSVMHEALHNHYTQKSLSLSPGK.

In addition to the above $WT_0$ with naturally paired light chains, three anti-GPC3×CD3 bispecific antibodies were designed to prevent mispairing of heavy and light chains: CM-C (GPC3 Fab × Crossmab-CD3), CM-G (Crossmab-GPC3 × CD3 Fab), and WT (GPC3 scFv × CD3 Fab).

The sequences of two heterologous heavy chains and two heterologous light chains of the CM-C bispecific antibody are shown in SEQ ID NO: 163, SEQ ID NO: 123, SEQ ID NO: 161, and SEQ ID NO: 121.

The sequences of two heterologous heavy chains and two heterologous light chains of the CM-G bispecific antibody are shown in SEQ ID NO: 119, SEQ ID NO: 167, SEQ ID NO: 117, and SEQ ID NO, SEQ ID NO: 165.

The sequences of two heterologous heavy chains and one light chain of the WT bispecific antibody had sequences as shown in SEQ ID NO: 119, SEQ ID NO: 159, and SEQ ID NO: 117.

The structural diagrams of these bispecific antibodies are illustrated in FIG. 2: CM-C and CM-G in part I, and WT in part II.

Four anti-GPC3×CD3 bispecific antibodies were designed as controls. The Fc regions of all control molecules containing OA/OB differ only in their antigen-binding arms. The details are summarized in Table 4.

Table 4. Heavy and Light Chain Sequences of Four Control Antibodies for anti-GPC3×CD3 Bispecific Antibodies

| Negative Control WT$_0$ | Triple-chain Positive Control WT |
|---|---|
| pCDNA3.1-CD3-LC (SEQ ID NO: 117) | pCDNA3.1-CD3-LC (SEQ ID NO: 117) |
| pFUSE-CD3-HC-OA (SEQ ID NO: 119) | pFUSE-CD3-HC-OA (SEQ ID NO: 119) |
| pCDNA3.1-GPC3-LC (SEQ ID NO: 121) | pFUSE-GPC3-scFv-OB (SEQ ID NO: 159) |
| pFUSE-GPC3-HC-OB (SEQ ID NO: 123) | |
| **CM-C** | **CM-G** |
| pCDNA3.1-CD3-VL-CH1 (SEQ ID NO: 161) | pCDNA3.1-CD3-LC (SEQ ID NO: 117) |
| pFUSE-CD3-VH-KCL-OA (SEQ ID NO: 163) | pFUSE-CD3-HC-OA (SEQ ID NO: 119) |
| pCDNA3.1-GPC3-LC (SEQ ID NO: 121) | pCDNA3.1-GPC3-VL-CH1 (SEQ ID NO: 165) |
| pFUSE-GPC3-HC-OB (SEQ ID NO: 123) | pFUSE-GPC3-VH-KCL-OB (SEQ ID NO: 167) |

SEQ ID NO: 163:

EVQLVESGGGLVQPGGSLKLSCAASGFTFNTYAMNWVRQAPGKGLEWV
ARIRSKYNNYATYYADSVKDRFTISRDDSKNTAYLQMNNLKTEDTAMYYCV
RHGNFGTSYVSWFAYWGQGTLVTVSSASVAAPSVFIFPPSDEQLKSGTASV
VCLLNNFYPREAKVQWKVDNALQSGNSQESVTEQDSKDSTYSLSSTLTLSK
ADYEKHKVYACEVTHQGLSSPVTKSFNRGECEPKSCDKTHTCPPCPAPEAA
GGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISK
AKGQPREPQVYTLPPSREEMTKNQVSLTCLVKGFYPSDIAVEWESNGQPE
NNYKTTKKKLDSDGSFFLYSKLTVDKSRWQQGNVFSCSVMHEALHNHYTQ
KSLSLSPGK;

SEQ ID NO: 161:

DIQMTQEPSLTTSPGGTVTLTCRSSTGAVTTSNYANWVQEKPGQAPRGLI
GGTNKRAPGTPARFSGSLIGGKAALTITGVQPEDEAIYFCALWYSNLWVFG
GGTKLEIKRSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTK
VDKKV;

SEQ ID NO: 167:

QVQLVQSGAEVKKPGASVKLSCKASGYTFTDYEMHWVKQTPGKGLKWI
GALDPKTGDTAYAQKFQGRATLTADTSTDTAYMELSSLRSEDSAVYYCTRF
YSYTYWGQGTLVTVSSASVAAPSVFIFPPSDEQLKSGTASVVCLLNNFYPRE
AKVQWKVDNALQSGNSQESVTEQDSKDSTYSLSSTLTLSKADYEKHKVYAC
EVTHQGLSSPVTKSFNRGECEPKSCDKTHTCPPCPAPEAAGGPSVFLFPPK
PKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVY
TLPPSREEMTKNQVSLTCLVKGFYPSDIAVEWESNGQPENNYKTDDDVLDS
DGSFFLYSKLTVDKSRWQQGNVFSCSVMHEALHNHYTQKSLSLSPGK;

SEQ ID NO: 165:

DIVLTQSPLSLTVTPGEPASISCRSSESLVHSNGNTYLSWLQQRPGQPPR
LLIYKISNRFSGVPDRFSGSGAGTDFTLKISRVEAEDVGVYYCVQVSSFPYTF
GQGTKVEIKSSASTKGPSVFPLAPSSKSTSGGTAALGCLVKDYFPEPVTVS
WNSGALTSGVHTFPAVLQSSGLYSLSSVVTVPSSSLGTQTYICNVNHKPSN
TKVDKKV;

SEQ ID NO: 159:

QVQLVQSGAEVKKPGASVKLSCKASGYTFTDYEMHWVKQTPGKGLKWI
GALDPKTGDTAYAQKFQGRATLTADTSTDTAYMELSSLRSEDSAVYYCTRF
YSYTYWGQGTLVTVSSGGGGSGGGGSGGGGSDIVLTQSPLSLTVTPGEPA
SISCRSSESLVHSNGNTYLSWLQQRPGQPPRLLIYKISNRFSGVPDRFSGSG
AGTDFTLKISRVEAEDVGVYYCVQVSSFPYTFGQGTKVEIK.

(2) Molecular Cloning of Expression Plasmid

The nucleotide sequence encoding the anti-CD3 light chain (SEQ ID NO: 117) and the anti-CD3 heavy chain (SEQ ID NO: 119) were synthesized. The anti-CD3 light chain sequence was cloned into the modified plasmid pCDNA3.1(+) (Invitrogen, Cat. No. V790-20) using standard molecular cloning methods (Sambrook JF, E. F. et al., Molecular cloning: a laboratory manual. 4th ed. Cold Spring Harbor Laboratory Press, Cold Spring Harbor, New York: 2012), and the expression plasmid was named pCDNA3.1-CD3-LC. Similarly, the anti-CD3 heavy chain was cloned into the plasmid pFUSE-hIgG1-Fc2 (InvivoGene), and the expression plasmid was named pFUSE-CD3-HC-OA.

The nucleotide sequence encoding the anti-GPC3 light chain (SEQ ID NO: 121) and the anti-GPC3 heavy chain (SEQ ID NO: 123) were synthesized. The anti-GPC3 light chain was cloned into the modified plasmid pCDNA3.1(+) using the standard molecular cloning methods so that it could express and secrete the antibody in cells (e.g., HEK293 cell). The expression plasmid was named pCDNA3.1-GPC3-LC. The anti-GPC3 heavy chain was cloned into the plasmid pFUSE-hIgG1-Fc2 (InvivoGene), and the expression plasmid was named pFUSE-GPC3-HC-OB.

(3) Modification of Bispecific Antibodies

The combinations of amino acid mutations at the CH1-CL binding interface shown in Table 3 were obtained by screening. From these combinations, two exemplary anti-GPC3×CD3 bispecific antibodies (11A10×8D6 and 7A10×11B7) were selected and introduced. These antibodies, derived from the mutations listed in Table 3, were named MutA and MutB (refer to Table 5).

For MutA, the anti-CD3 heavy chain sequence of the bispecific antibody $WT_0$ was modified by introducing the mutations T106H, S186T, S188Y, and V190F mutations. The anti-CD3 light chain sequence was modified by introducing the mutations S174A, S176E, and T178A mutations. The anti-GPC3 heavy chain sequence was modified by introducing the mutations S186M, S188I, and V190W mutations. The anti-GPC3 light chain sequence was modified by introducing the mutations S174R, S176H, and T178V mutations.

For MutB, the anti-CD3 heavy chain sequence of the bispecific antibody $WT_0$ was modified by introducing the mutations T106H, S186V, S188L, and V190M mutations. The anti-CD3 light chain sequence was modified by introducing the mutations S174R, S176W, and T178L mutations. The anti-GPC3 heavy chain sequence was modified by introducing the mutations S186T, S188R, and V190F mutations.

### Table 5. Mutated Combination I of Anti-GPC3×CD3 Bispecific Antibodies

| Plasmid / Mutated Combination | MutA | MutB |
|---|---|---|
| pFUSE-CD3-HC-OA | T106H, S186T, S188Y, V190F | T106H, S186V, S188L, V190M |
| pCDNA3.1-CD3-LC | S174A, S176E, T178A | S174R, S176W, T178L |
| pFUSE-GPC3-HC-OB | S186M, S188I, V190W | S186T, S188R, V190F |
| pCDNA3.1-GPC3-LC | S174R, S176H, T178V | $WT_0$ |

To enhance the specificity of the light chains for the heavy chains and prevent mispairing, amino acid mutations were introduced into the antibody sequences based on incorporating charge mutations and considering the size of the amino acids. The resulting mutated combinations, MutA+D-D and MutB+K-K of the anti-GPC3×CD3 bispecific antibodies are detailed in Table 6. MutA+D-D was derived from MutA, where an A178D mutation was introduced to the anti-CD3 light chain sequence, and an I188D mutation was introduced to the anti-GPC3 heavy chain. MutB+K-K was derived from MutB, where an L178K mutation was introduced to the anti-CD3 light chain, and a T192K mutation was introduced to the anti-GPC3 heavy chain.

### Table 6. Mutated Combination II of Anti-GPC3×CD3 Bispecific Antibodies

| Plasmid / Mutated Combination | MutA+D-D | MutB+K-K |
|---|---|---|
| pFUSE-CD3-HC-OA | T106H, S186T, S188Y, V190F | T106H, S186V, S188L, V190M |
| pCDNA3.1-CD3-LC | S174A, S176E, A178D | S174R, S176W, L178K |
| pFUSE-GPC3-HC-OB | S186M, I188D, V190W | S186T, S188R, V190F, T192K |
| pCDNA3.1-GPC3-LC | S174R, S176H, T178V | $WT_0$ |

### Example 4. Expression and Purification of Bispecific Antibodies

(1) Expression of Anti-GPC3×CD3 Bispecific Antibodies

Plasmids were extracted and isolated using the Endo-Free-Plasmid Maxi Kit (100) (purchased from OMEGA, catalog No. D6926-04), following the provided kit instructions. The expression vectors constructed in Example 1, namely the mutations MutA, MutB, MutA+D-D, and MutB+K-K listed in Table 5 and Table 6, were co-transfected into cells. The suspension culture of transfected cell was incubated in dark at 37 °C, with 5% $CO_2$ and 120 rpm on a shaking incubator for 4 to 5 days.

(2) Purification of Anti-GPC3×CD3 Bispecific Antibodies

Expression supernatants were harvested by centrifugation and filtered with a 0.22 $\mu$m filter. The bispecific antibody in the supernatants were captured using pre-equilibrated Protein-A affinity chromatography resin (MabSelect-SuReTM, purchased from GE Healthcare, Cat. No. 17-5438-02). Subsequently, five column volumes were eluted using elution buffer, and neutralized to pH7.0. The eluted samples were analyzed by SDS-PAGE.

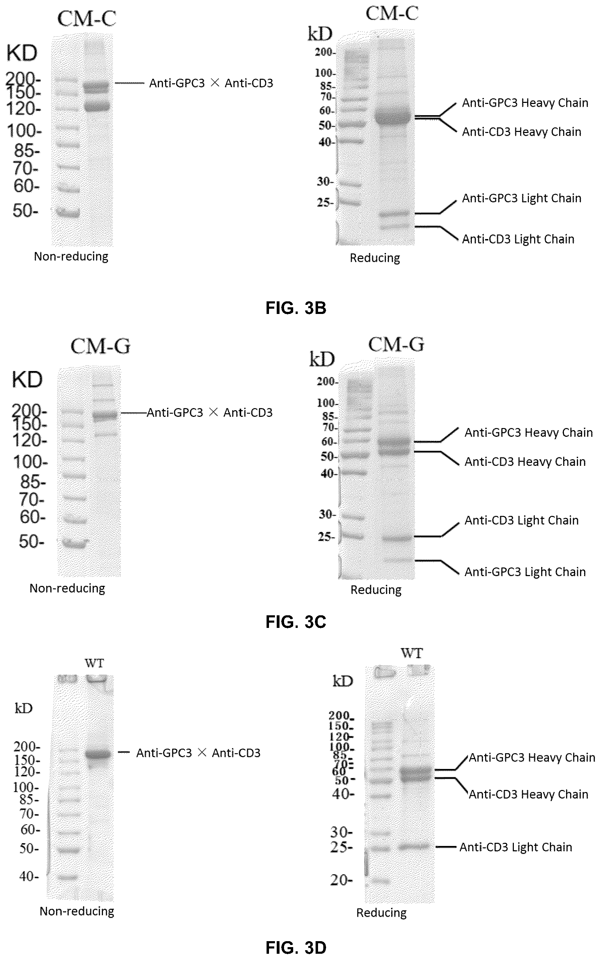

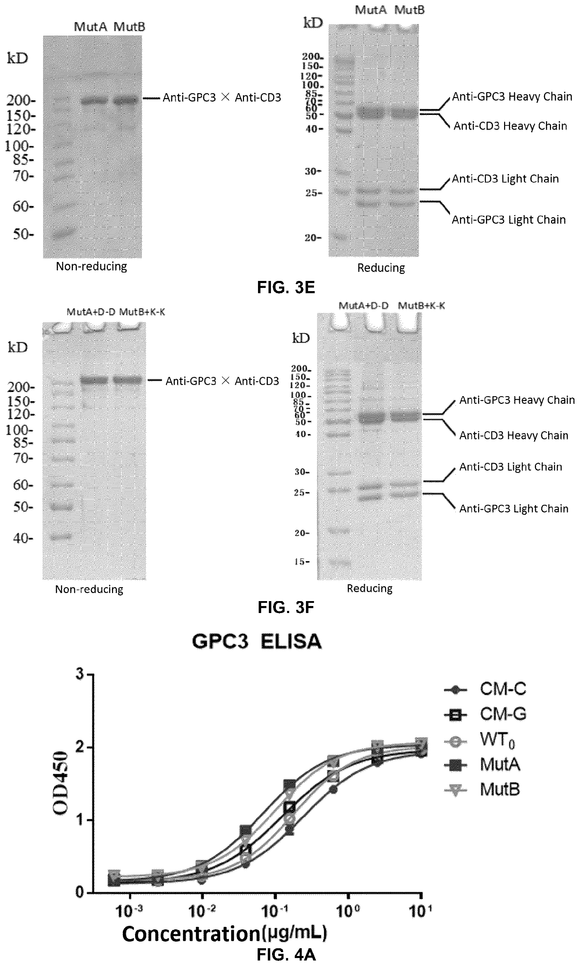

The purities of the target proteins purified by protein A, for the respective control bispecific antibodies $WT_0$, CM-C, CM-G, and WT, were shown in FIG. 3A to FIG. 3D. Here, $WT_0$ served as a negative control, CM-C was a Crossmab-CD3 arm of anti-GPC3×CD3 bispecific antibody , CM-G was a Crossmab-GPC3 arm of anti-GPC3×CD3 bispecific antibody and WT was a triple-chain positive control antibody without heavy/light chain mispairing. The results revealed that the heavy chains of the bispecific antibodies paired successfully at an equimolar ratio, resulting in heterodimer formation. This was confirmed by the 1:1 expression ratio of the heterologous heavy chains observed in reducing SDS-PAGE, which was a result of the OA/OB mutations present in the Fc region of all combinations. However, a notable difference was observed in the pairing ratio of the two heterologous light chains. Among the negative controls $WT_0$, Crossmab-CD3, and Crossmab-GPC3 (FIG. 3A, FIG. 3B, and FIG. 3C), the variation in the light chain pairing ratio indicated ineffective pairing with the corresponding heavy chains, thus failing to produce the target bispecific antibodies effectively. Conversely, the MutA, MutB, MutA+D-D, and MutB+K-K molecules (FIG. 3E and FIG. 3F) demonstrated effective pairing of light chains with their corresponding heavy chains, indicating effective generation of the desired bispecific antibody.

**Example 5. Characterization of Antigen-Binding Affinity in Bispecific Antibodies**

To validate the antigen-binding abilities of bispecific antibodies, which have been engineered with site-specific charge mutations based on amino acid size as detailed herein, an ELISA assay was conducted to evaluate their binding activity with CD3 and GPC3 antigens. The protocol is as follows.

(1) The coated antigens were human GPC3 antigen (Acrobiosystems Inc.) and human CD3 antigen (Sino Biological Inc.).

(2) Coating: Dilute the antigen with $1 \times$ PBS. Apply 200 $\mu$L of the diluted antigens to an ELISA plate. Subsequently, seal the reaction wells with a plate-sealing film and coated at room temperature for 1.5 hours or overnight at 4 °C. Wash the plate six times with 0.05% PBST.

(3) Blocking: Prepare 3% M-PBS using skimmed milk powder and PBS buffer. Add 300 $\mu$L of 3% MPBS to each well of the ELISA plate. Seal the reaction wells with a plate-sealing film and incubate at room temperature for 1 hour. Wash the plate six times with 0.05% PBST.

(4) Antibody Binding: Add 100 $\mu$L of antibodies diluted to gradient concentrations. Perform experiments with two replicate wells per antibody concentration. After adding the antibodies, seal the reaction wells with a plate-sealing film and incubate at room temperature for 1.5 hours. Wash the plate six times with 0.05% PBST.

(5) Secondary Antibody: Dilute the HRP-conjugated goat anti-human IgG secondary antibody (Peprotech, Catalog No. SA0001-17) to a 1:2000 ratio in 3% M-PBS solution. Add the diluted secondary antibody to the wells. Seal the reaction wells with a plate-sealing film and incubate at room temperature for 1 hour. Wash the plate six times with 0.05% PBST.

(6) Color Reaction: Add 100 $\mu$L of TMB substrate solution and incubate at room temperature in the dark for 3 to 5 min.

(7) Stop: Add 100 $\mu$L of stopping solution (1 M HCl), mix thoroughly, and measure the OD at 450 nm.

(8) Data Analysis: Process the data using GraphPad Prism 5 software. Generate dose-response curves by plotting log (sample concentration) on the x-axis and the OD value on the y-axis, and calculate $EC_{50}$ to represent the ability of the samples to bind to the antigens.

Table 7 showed the binding affinities of the anti-GPC3$\times$CD3 bispecific antibodies MutA and MutB to the negative control antibodies $WT_0$, CM-C, and CM-G with respect to the GPC3 and CD3 antigens.
Table 8 showed the binding affinities of the GPC3$\times$CD3 bispecific antibodies MutA+D-D and MutB+K-K, as well as the negative controls $WT_0$, CM-C, and CM-G, for their binding with the GPC3 and CD3 antigens.
Table 9 showed the binding affinities of the GPC3$\times$CD3 bispecific antibodies MutA and MutB , as well as the positive control antibody WT, for their binding with the GPC3 and CD3 antigens.
Table 10 showed the binding affinities of the anti-GPC3$\times$CD3 bispecific antibodies MutA+D-D and MutB+K-K , as well as the positive control antibody WT, for their binding with the GPC3 and CD3 antigens.

## Table 7

| $EC_{50}$ \ Sample | CM-C | CM-G | $WT_0$ | MutA | MutB |
|---|---|---|---|---|---|
| GPC3-$EC_{50}$ | 0.2381 | 0.1242 | 0.1811 | 0.06815 | 0.1019 |
| CD3-$EC_{50}$ | 7.839 | 0.2983 | 0.4371 | 0.1235 | 0.1408 |

## Table 8

| EC$_{50}$ \ Sample | CM-C | CM-G | WT$_0$ | MutA+D-D | MutB+K-K |
|---|---|---|---|---|---|
| GPC3-EC$_{50}$ | 0.12562 | 0.09648 | 0.1028 | 0.02081 | 0.02123 |
| CD3-EC$_{50}$ | 15.86 | 0.1971 | 0.7029 | 0.1290 | 0.1741 |

## Table 9

| EC$_{50}$ \ Sample | WT | MutA | MutB |
|---|---|---|---|
| GPC3-EC$_{50}$ | 0.02658 | 0.03254 | 0.03879 |
| CD3-EC$_{50}$ | 0.08086 | 0.1470 | 0.1681 |

## Table 10

| EC$_{50}$ \ Sample | WT | MutA+D-D | MutB+K-K |
|---|---|---|---|
| GPC3-EC$_{50}$ | 0.02836 | 0.02981 | 0.03057 |
| CD3-EC$_{50}$ | 0.04254 | 0.05915 | 0.06105 |

FIG. 4A to FIG. 4D showed the binding affinities of the bispecific antibodies MutA, MutB, MutA+D-D and MutB+K-K, as well as the negative controls WT$_0$, CM-C, and CM-G, for interactions with the GPC3 and CD3 antigens.

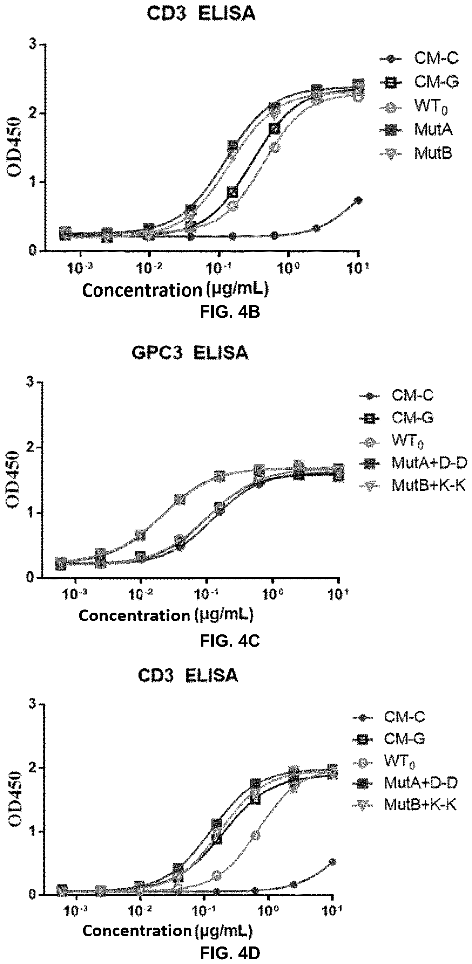

FIG. 4A and FIG. 4B indicate that MutA and MutB exhibit superior affinities for the two targeted antigens, demonstrating a significant advantage over the negative controls WT$_0$, CM-C, and CM-G.

FIG. 4C and FIG. 4D demonstrate that the modified bispecific antibodies MutA+D-D and MutB+K-K exhibit enhanced binding affinities for the two targeted antigens, significantly outperforming the negative controls WT$_0$, CM-C, and CM-G.

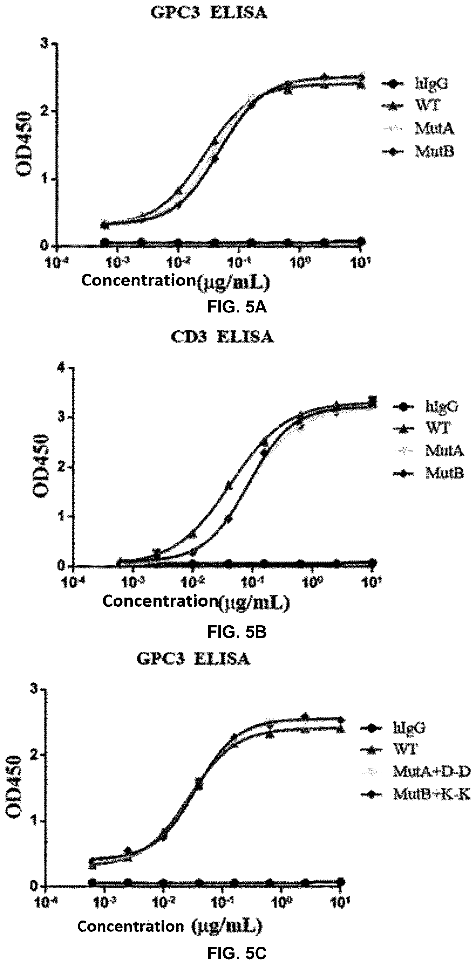

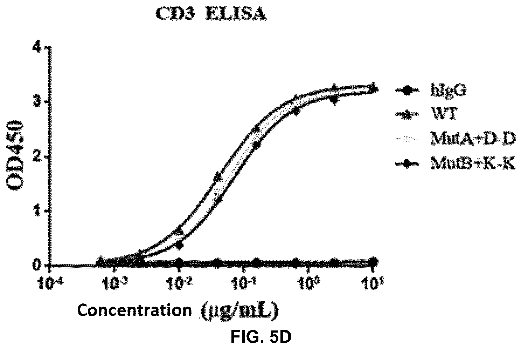

FIG. 5A to FIG. 5D illustrate the binding affinities of the bispecific antibodies MutA, MutB, MutA+D-D, and MutB+K-K, as well as the positive control antibody WT, for interactions with the GPC3 and CD3 antigens. The results indicate that MutA, MutB, MutA+D-D, and MutB+K-K display binding activities to the two targeted antigens comparable to the positive control antibody WT. This finding suggests that there is no significant mispairing in the construction of these molecules.

The above results demonstrated that, compared to the negative controls WT$_0$, CM-C and CM-G and the positive control antibody WT, the anti-GPC3×CD3 bispecific antibodies MutA, MutB, MutA+D-D, and MutB+K-K in the invention exhibit stronger binding activity to GPC3 and CD3 antigens than the negative controls WT$_0$, CM-C, and CM-G. The binding activity of these inventive antibodies is superior to that of the negative controls and is found to be equivalent to the positive control antibody WT, without the occurrence of light chain mispairing. This indicates that the modified bispecific antibodies retain their antigen-binding capability, as enhanced by the methods of the present invention.

## Example 6. Verification of Binding Activity of Tetra-chain Bispecific Antibodies by Flow Cytometry

To further study the binding activity of the anti-GPC3×CD3 bispecific antibodies towards GPC3, the present invention utilizes flow cytometry to assess their binding affinity to GPC3-positive cells.

First, the GPC3 gene was cloned into a pcDNA3.1 vector, and transformed into E. coli, and amplified. Plasmids were then extracted using a Maxi Kit (OMEGA), following the manufacturer's protocol. The above plasmids were transfected into 293F cells using PEI as the transfection reagent. The transfected cells, named 293F/GPC3 cells, transiently expressed GPC3, while 293F cells were used as a negative control.

293F and 293F/GPC3 cells were collected and washed once with 2% FBS/PBS (diluent) pre-cooled at 4 °C, and then the cells were resuspended in an appropriate amount of a diluent to achieve a density of $5.0×10^6$ cells/mL. The cells were inoculated into a 96-well plate at 100 μL per well, i.e., inoculated in an amount of $5.0×10^5$ cells per well. A anti-GPC3×CD3 bispecific antibody was prepared in diluent to a concentration of 5 μg/mL. 100 μL of the antibody solution was mixed with an equal volume of either 293F or 293F/GPC3 cells to achieve a final concentration of 2.5 μg/mL. The mixture was incubated in a 96-well plate at 2 to 8 °C for 1 h. Subsequently, the 96-well plate was centrifuged at 500×g at 4°C for 5 min, the supernatant was removed, and the cells were washed twice with 200 μL of diluent. The cells were resuspended in a diluent containing PE anti-human IgG FC (Invitrogen) and then incubated at 2 to 8 °C for 1 h in the dark. The 96-well plate was centrifuged at 500×g at 4°C for 5 min, the supernatant was removed, and the cells were washed twice with a 200 μL of diluent. The cells were resuspended in a 200 μL of diluent and then immediately analyzed using a flow cytometer.

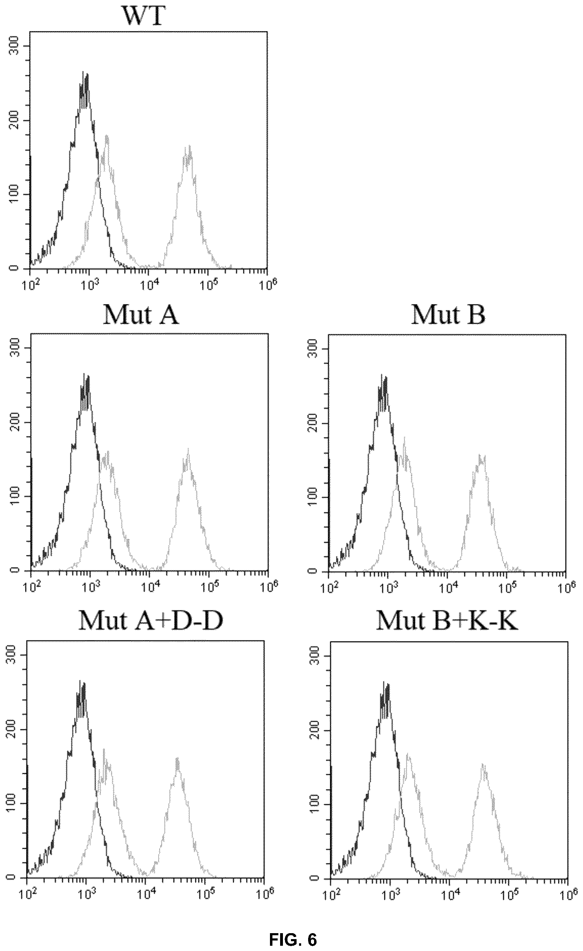

As shown in FIG. 6, the anti-GPC3×CD3 bispecific antibodies MutA, MutB, MutA+D-D, and MutB+K-K all demonstrated high binding activity to GPC3, and their binding activity was consistent with that of the triple-chain positive control antibody (WT), which does not exhibit light chain mispairing.

## Example 7. Characterization of T Cell Activation of Bispecific Antibodies

The T cell activation activity of the anti-GPC3×CD3 bispecific antibodies was assessed by using a detection system comprising a Jurkat/NFAT-Luc reporter cell line and GPC3-positive human hepatoma cells (HepG2 cells).

The Jurkat/NFAT-Luc cell line was used as effector cells, with luciferase genes regulated by NFAT (Nuclear factor of activated T cells) transcription factors. The HepG2 cells (GPC3-positive) were used as target cells. In the absence of anti-GPC3×CD3 bispecific antibodies, co-culture of Jurkat/NFAT-Luc cells with HepG2 cells did not result in luciferase expression.

However, upon the introduction of the anti-GPC3×CD3 bispecific antibody, the engagement of GPC3 on HepG2 cells with the CD3 signaling pathway in Jurkat/NFAT-Luc cells was facilitated. This interaction promoted the expression of luciferase, thereby enabling the assessment of Jurkat T cell activation levels by measuring the luminescence emitted due to luciferase expression.

In the experiment, HepG2 cells ($4.0 \times 10^5$) and Jurkat/NFAT-Luc cells ($4.0 \times 10^6$) were sequentially added to a 96-well cell culture plate, each in a volume of 50 μL. Then, the anti-GPC3×CD3 bispecific antibodies were diluted to concentrations of 30 μg/mL, 7.5 μg/mL, 1.875 μg/mL, 0.4688 μg/mL, 0.1172 μg/mL, 0.0293 μg/mL, 0.0073 μg/mL, 0.0018 μg/mL, 0.0005 μg/mL, and 0.0001 μg/mL. These antibodies were added to the cell culture plate described above, stationarily cultured at 37 °C for 5 h, and then a ONE-Glo Luciferase detection reagent was added thereto, and chemiluminescence values were measured using a multifunctional microplate reader. A four-parameter fitting was conducted with the logarithm of antibody concentration on the x-axis and average chemiluminescence values on the y-axis. Dose-response curves were plotted to determine the EC50 value for each curve. The EC50 value represents the T cell activation potency of the anti-GPC3×CD3 tetra-chain bispecific antibodies.

Table 11 showed the comparison of the T cell activation activity between the anti-GPC3×CD3 bispecific antibodies MutA, MutB and the negative control antibodies $WT_0$, CM-C, and CM-G,

Table 12 showed the comparison of the T cell activation activity between the anti-GPC3×CD3 bispecific antibodies MutA+D-D, MutB+K-K and the negative controls $WT_0$, CM-C, and CM-G,

Table 13 showed the comparison of T cell activation activity between the anti-GPC3×CD3 bispecific antibodies MutA, MutB and the triple-chain positive control antibody WT,

and Table 14 showed the comparison of the T cell activation activity between the anti-GPC3×CD3 bispecific antibodies MutA+D-D, MutB+K-K and the triple-chain positive control antibody WT.

## Table 11

| Sample / $EC_{50}$ | CM-C | CM-G | $WT_0$ | MutA | MutB |
|---|---|---|---|---|---|
| $EC_{50}$ | 3.396 | 0.1209 | 0.1069 | 0.04162 | 0.04936 |

## Table 12

| Sample / $EC_{50}$ | CM-C | CM-G | $WT_0$ | MutA+D-D | MutB+K-K |
|---|---|---|---|---|---|
| $EC_{50}$ | 14.15 | 0.08178 | 0.08993 | 0.01308 | 0.01629 |

## Table 13

| Sample / $EC_{50}$ | WT | MutA | MutB |
|---|---|---|---|
| $EC_{50}$ | 0.02601 | 0.04626 | 0.03920 |

## Table 14

| Sample / EC$_{50}$ | WT | MutA+D-D | MutB+K-K |
|---|---|---|---|
| EC$_{50}$ | 0.009699 | 0.01304 | 0.01096 |

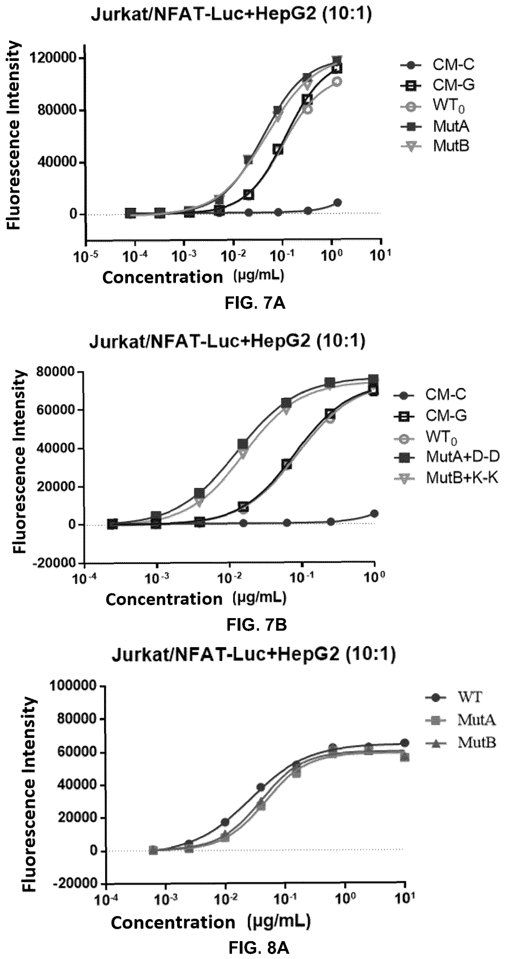

FIG. 7A and FIG. 7B showed the comparison of the T cell activation activity between MutA, MutB, MutA+D-D, and MutB+K-K with that of the negative controls WT$_0$, CM-C, and CM-G. The results showed that MutA, MutB, MutA+D-D, and MutB+K-K had higher T cell activation activity than the negative controls WT$_0$, CM-C, and CM-G.

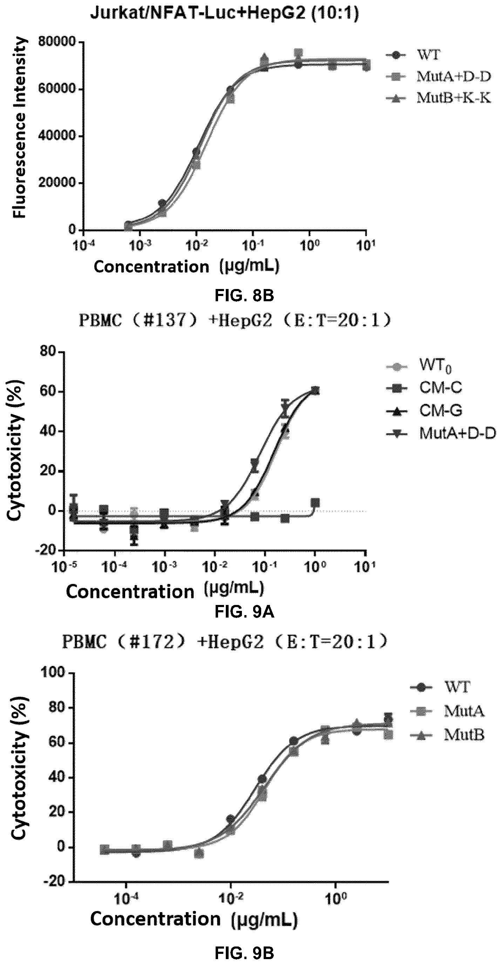

FIG. 8A and FIG. 8B showed the comparison of the T cell activation activity between MutA, MutB, MutA+D-D and MutB+K-K with that of the positive control WT, which was a triple-chain bispecific antibody without mispairing of light chains. The results showed that the T cell activation activity of MutA, MutB, MutA+D-D, and MutB+K-K was consistent with that of the triple-chain positive control antibody WT, confirming the absence of light chain mispairing.

The above results demonstrated that when co-incubated with GPC3-positive tumor cells HepG2 and Jurkat/NFAT-Luc cells, the anti-GPC3×CD3 tetra-chain bispecific antibodies MutA, MutB, MutA+D-D, and MutB+K-K disclosed herein exhibited superior activation activity on Jurkat T cells than the negative controls WT$_0$, CM-C, and CM-G. Furthermore, their activation activity were consistent with that of the positive control WT, a triple-chain bispecific antibody without light chains mispairing.

## Example 8. Characterization of T Cell-Mediated Tumor Killing Assay by Bispecific Antibodies

The CD3-binding arm of the anti-GPC3×CD3 bispecific antibody specifically targets CD3 complexes on the surface of T cells, while the GPC3-binding arm specifically binds to GPC3 antigens present on the surface of tumor cells. This simultaneous combination of properties facilitates immunological bridging between T cells and tumor cells. As a result, T cells are activated to release cell-killing proteins, such as perforin and granzyme B, which effectively eliminate the tumor cells.

When the membranes of tumor cells were damaged, the permeability of the cell membranes increased, leading to the release of lactate dehydrogenase (LDH) from the cytoplasm into the culture supernatant. A measured volume of the supernatant was aliquoted, and lactic acid was introduced as a substrate for lactate dehydrogenase. The enzyme then catalyzed a dehydrogenation reaction, resulting in the formation of the chromogenic product formazan. This product exhibits a characteristic absorption peak at a wavelength of 490 nm. A cell supernatant with higher lactate dehydrogenase content exhibited a darker color and had a higher absorbance value. Therefore, the absorbance value could be measured to quantify the lactate dehydrogenase released from tumor cells, allowing for the calculation of the tumor cell-killing activity of PBMCs mediated by the anti-GPC3 × CD3 bispecific antibodies.

The killing rate was calculated by the following formula:

$$\text{cytotoxicity (\%)} = (OD_{sample}-SR)/(MR-SR) \times 100\%$$

where SR = OD$_{spontaneous}$ release well (target cell + effector cell), MR = OD$_{maximum}$ release well (target cell).

(1) Preparation of PBMCs

Peripheral blood mononuclear cells (PBMCs) are cells in peripheral blood with a single nucleus. They include lymphocytes, monocytes, dendritic cells, and a small number of other cells such as hematopoietic stem cells. Relative pure PBMCs can be obtained by separating them using the Ficoll centrifugation method. This involves extracting the middle buffy coat after centrifugation, followed by multiple rounds of centrifugation and washing. The PBMCs were then adjusted to a density of $2.00 \times 10^6$ cells/mL with phenol red-free RPMI 1640 (containing 5% FBS) for subsequent use.

(2) Preparation of Target Cells

GPC3-positive target cells used in the present invention were human hepatoma cells HepG2. To ensure that the target cells are in optimal growth status, subculture should be performed 1 to 2 days before the experiment. The well-grown HepG2 cells are obtained by removing the culture medium, washing the culture flask twice with 1× PBS (pH 7.4), and then adding 2 mL of 0.05% trypsin-EDTA for digesting at 37 °C. The digestion was terminated with the corresponding culture medium, and the cells were collected by horizontal centrifugation at 1,000 rpm (190×g) for 5 min. After being washed twice with 1 × PBS (pH 7.4), the cells were adjusted to a concentration of $2.00 \times 10^5$ cells/mL with phenol red-free RPMI 1640 complete medium for further use.

(3) Preparation of Bispecific Antibody Samples

Bispecific antibody was initially diluted to 30 μg/mL with 1× PBS buffer (pH 7.4) and then gradient diluted 4-fold to a total of 10 gradient concentrations of 30 μg/mL, 7.5 μg/mL, 1.875 μg/mL, 0.4688 μg/mL, 0.1172 μg/mL, 0.0293 μg/mL, 0.0073 μg/mL, 0.0018 μg/mL, 0.0005 μg/mL, and 0.0001 μg/mL.

(4) Incubation and Detection

A 96-well cell culture plate was prepared, to which target cells (50 μL per well), PBMCs (100 μL per well), and the gradient-diluted samples (50 μL perwell) were added sequentially, ensuring a uniform mixture and a PBMC to target cell ratio of 20:1. The initial concentration of the samples was 10 μg/mL. The control wells were supplemented with 200 μL of phenol red-free RPMI 1640 medium. The culture plate was incubated in a 37 °C, 5% $CO_2$ incubator. The cell-killing toxicity was detected using a lactate dehydrogenase cytotoxicity assay kit. Dose-response curves were drawn by four-parameter fitting using the logarithmic value of the antibody concentration as the abscissa and the killing activity as the ordinate, and the $EC_{50}$ values of the respective curves were obtained to represent the target cell-killing activity of the anti-GPC3×CD3 bispecific antibodies.

Table 15 showed the comparison of cytotoxic activity between the anti-GPC3×CD3 bispecific antibody MutA+D-D and the negative controls $WT_0$, CM-C, and CM-G on killing of HepG2 cells,

Table 16 showed the comparison of cytotoxic activity between the anti-GPC3×CD3 bispecific antibodies MutA, MutB, and the positive control WT(a triple-chain bispecific antibody) on killing of HepG2,

and Table 17 showed the comparison of cytotoxic activity between the anti-GPC3×CD3 bispecific antibodies MutA+D-D, MutB+ K-K, and the triple-chain positive control antibody WT on killing of HepG2 cells.

## Table 15

| $EC_{50}$ \ Sample | CM-C | CM-G | $WT_0$ | MutA+D-D |
|---|---|---|---|---|
| $EC_{50}$ | 1.167 | 0.1748 | 0.1962 | 0.05869 |

## Table 16

| $EC_{50}$ \ Sample | WT | MutA | MutB |
|---|---|---|---|
| $EC_{50}$ | 0.02861 | 0.04352 | 0.04934 |

## Table 17

| $EC_{50}$ \ Sample | WT | MutA+D-D | MutB+K-K |
|---|---|---|---|
| $EC_{50}$ | 0.02801 | 0.03038 | 0.04189 |

FIG. 9A showed the comparison of tumor cytotoxic activity between MutA+D-D and the negative controls $WT_0$, CM-C, and CM-G.

FIG. 9B showed the comparison of tumor cytotoxic activity between MutA, MutB, and the positive control WT (a triple-chain bispecific antibody).

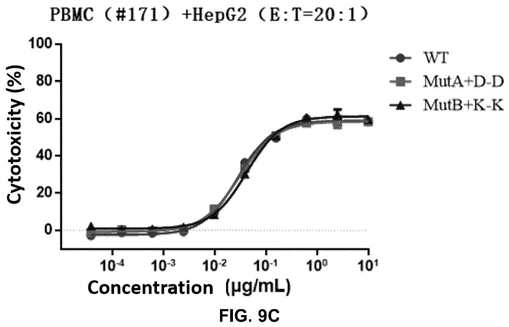

FIG. 9C showed the comparison of tumor cytotoxic activity between MutA+D-D, MutB+K-K, and the triple-chain positive antibody WT.

FIG. 9A showed that MutA+D-D had higher tumor cytotoxic activity than the negative controls $WT_0$, CM-C, and CM-G. FIGS.

FIGS. 9B and 9C demonstrate that the tumor cytotoxic activities of the bispecific antibodies MutA, MutB, MutA+D-D, and MutB+K-K are comparable to those of the positive control WT, a triple-chain bispecific antibody avoid light chain mispairing. This further indicated that the issue of mispairing between the two different light chains was effectively addressed through the modification of the four chains of bispecific antibodies in the present invention.

The above results demonstrated that, as compared with the negative controls $WT_0$, CM-C, and CM-G and the positive control WT (a triple-chain bispecific antibody), the anti-GPC3×CD3 tetra-chain bispecific antibodies MutA, MutB, MutA+D-D, and MutB+K-K disclosed in the present invention had better effects on killing HepG2 cells with high GPC3 expression than the negative controls $WT_0$, CM-C, and CM-G. This comparison further substantiates that the modifications according to the present invention effectively mitigate the issue of mispairing between the two distinct light chains.

In summary, the present invention encompasses a method whereby the probability of heavy and light chain mispairing within an antibody is significantly diminished through targeted amino acid mutations at the CH1-heavy chain and CL-light chain interface. This approach not only enhances the binding affinity to specific antigens but also augments the yield of the desired antibody construct. The anti-GPC3×CD3 bispecific antibodies, MutA, MutB, MutA+D-D and MutB+K-K, exhibit superior binding affinity to their targeted antigens, and demonstrate enhanced T cell activation and robust tumor cytotoxic activity, thereby presenting extensive application prospects.

The applicant declares that the detailed methods of the present invention are illustrated by means of the above examples in the present invention, but the present invention is not limited to the above detailed methods. In other words, it does not mean that the implementation of the present invention should rely on the above detailed methods. It will be appreciated by those skilled in the art that any improvements on the present invention, any equivalent replacements of respective raw materials of the products of the present invention, any additions of auxiliary ingredients, any selections of specific methods, and so on are within the scope of protection and invention of the present invention.

**Industrial Application**

The present invention provides bispecific antibodies and use thereof. In the present invention, by mutating amino acids at the interfaces between heavy chains CH1 and light chains CL of the antibodies or antibody fragments in terms of amino acid size and charge, the correct pairing of the heavy/light chains against different antigens is enhanced, their ability to bind to specific antigens is maintained, mispairing is reduced and their binding specificity is improved, and the yields of the target products are improved. Therefore, the present invention has excellent industrial applicability.

**Claims**

1. An antibody or antibody fragment, **characterized in that** the antibody or antibody fragment is a bispecific antibody or antibody fragment having a first heavy chain and a first light chain as well as a second heavy chain and a second light chain;

   wherein the first Fab region comprises a first heavy chain and a first light chain;
   the second Fab region comprises a second heavy chain and a second light chain;
   at least one, or a combination of at least two mutations, from the following set is introduced into the first Fab region of the antibody or antibody fragment:

   a) S186A/S186T/S186E/S186Q/S186M/S186D/S186K/S186V/S186W /S186Y in a heavy chain constant domain CH1; and
   T178M/T178Y/T178L/T178I/T178V/T178V/T178S/T178R/T178E in a light chain constant domain CL;
   b) S188Y/S188M/S188T/S188F/S188V/S188L/S188I/S188R in the heavy chain constant domain CH1; and
   S176M/S176L/S176Y/S176A/S176I/S176E/S176T/S176W/S176H/S17 6V in the light chain constant domain CL; and
   c) V190T/V190I/V190W/V190M/V190Y/V190F/V190L/V190Q/V190S in the heavy chain constant domain CH1; and
   S174F/S174N/S174M/S174A/S174T/S174W/S174R/S174C/S174K/S1 74H/S174Y/S174L in the light chain constant domain CL; and

   at least one mutation, or a combination of at least two mutations, from the following set is introduced into the second Fab region of the antibody or antibody fragment:

   a) S186A/S186T/S186E/S186Q/S186M/S186D/S186K/S186V/S186W /S186Y in a heavy chain constant domain CH1; and
   T178M/T178Y/T178L/T178I/T178A/T178V/T178S/T178R/T178E in a light chain constant domain CL;
   b) S188Y/S188M/S188T/S188F/S188V/S188L/S188I/S188R in the heavy chain constant domain CH1; and
   S176M/S176L/S176Y/S176A/S176I/S176E/S176T/S176W/S176H/S17 6V in the light chain constant domain CL; and
   c) V190T/V190I/V190W/V190M/V190Y/V190F/V190L/V190Q/V190S in the heavy chain constant domain CH1; and
   S174F/S174N/S174M/S174A/S174T/S174W/S174R/S174C/S174K/S1 74H/S174Y/S174L in the light chain constant domain CL.

2. The antibody or antibody fragment according to claim 1, wherein any one mutation of following sets are introduced into the first Fab region of the antibody or antibody fragment:

   a) S186T in the heavy chain constant domain CH1 and T178A in the light chain constant domain CL;
   b) S188Y in the heavy chain constant domain CH1 and S176E in the light chain constant domain CL; and
   c) V190F in the heavy chain constant domain CH1 and S174A in the light chain constant domain CL;

   or

   a) S186V in the heavy chain constant domain CH1 and T178L in the light chain constant domain CL;
   b) S188L in the heavy chain constant domain CH1 and S176W in the light chain constant domain CL; and
   c) V190M in the heavy chain constant domain CH1 and S174R in the light chain constant domain CL;

   or

   a) S186W in the heavy chain constant domain CH1 and T178M in the light chain constant domain CL;
   b) S188F in the heavy chain constant domain CH1 and S176V in the light chain constant domain CL; and
   c) V190T in the heavy chain constant domain CH1 and S174K in the light chain constant domain CL;

   or

   a) S186Y in the heavy chain constant domain CH1;
   b) S188M in the heavy chain constant domain CH1 and S176V in the light chain constant domain CL; and
   c) V190F in the heavy chain constant domain CH1;

   preferably, any one mutation of following sets is introduced into the second Fab region of the antibody or antibody fragment:

   a) S186M in the heavy chain constant domain CH1 and T178V in the light chain constant domain CL;
   b) S188I in the heavy chain constant domain CH1 and S176H in the light chain constant domain CL; and
   c) V190W in the heavy chain constant domain CH1 and S174R in the light chain constant domain CL;

   or

   a) S186T in the heavy chain constant domain CH1 and T178V in the light chain constant domain CL;
   b) S188R in the heavy chain constant domain CH1; and
   c) V190F in the heavy chain constant domain CH1;

   or

   a) S186K in the heavy chain constant domain CH1 and T178E in the light chain constant domain CL;
   b) S188L in the heavy chain constant domain CH1 and S176M in the light chain constant domain CL; and
   c) S174Y in the light chain constant domain CL;

   or

   a) S186M in the heavy chain constant domain CH1 and T178V in the light chain constant domain CL;
   b) S188I in the heavy chain constant domain CH1 and S176H in the light chain constant domain CL; and
   c) V190W in the heavy chain constant domain CH1 and S174R in the light chain constant domain CL.

3. The antibody or antibody fragment according to claim 1 or 2, wherein an A178D mutation is further introduced into the light chain constant domain CL of the first Fab region, and an I188D mutation is further introduced into the heavy chain constant domain CH1 of the second Fab region;

   preferably, an L178K mutation is further introduced into the light chain constant domain CL of the first Fab region, and a T192K mutation is further introduced into the heavy chain constant domain CH1 of the second Fab region; and
   preferably, mutations introduced into the first Fab region and the second Fab region of the antibody or antibody fragment are the same or different.

4. A bispecific antibody, **characterized in that** the bispecific antibody comprises the antibody or antibody fragment according to any of claims 1 to 3,

wherein preferably, the light chain constant domain CL of the bispecific antibody are each independently selected from any one or more of the following sequences: SEQ ID NO: 3, SEQ ID NO: 7, SEQ ID NO: 11, SEQ ID NO: 15, SEQ ID NO: 19, SEQ ID NO: 23, SEQ ID NO: 27, SEQ ID NO: 31, SEQ ID NO: 35, SEQ ID NO: 39, SEQ ID NO: 43, and SEQ ID NO: 47, SEQ ID NO: 51, SEQ ID NO: 55, SEQ ID NO: 59, SEQ ID NO: 63, SEQ ID NO: 67, SEQ ID NO: 71, SEQ ID NO: 75, SEQ ID NO: 79, SEQ ID NO: 83, SEQ ID NO: 87, SEQ ID NO: 91, SEQ ID NO: 95, SEQ ID NO: 99, SEQ ID NO: 103, SEQ ID NO: 107, SEQ ID NO: 111, and SEQ ID NO: 115; preferably, the heavy chain constant domain CH1 of the bispecific antibody are each independently selected from any one or more of the following sequences: SEQ ID NO: 1, SEQ ID NO: 5, SEQ ID NO: 9, SEQ ID NO: 13, SEQ ID NO: 17, SEQ ID NO: 21, SEQ ID NO: 25, SEQ ID NO: 29, SEQ ID NO: 33, SEQ ID NO: 37, SEQ ID NO: 41, SEQ ID NO: 45, SEQ ID NO: 49, SEQ ID NO: 53, SEQ ID NO: 57, SEQ ID NO: 61, SEQ ID NO: 65, SEQ ID NO: 69, SEQ ID NO: 73, SEQ ID NO: 77, SEQ ID NO: 81, SEQ ID NO: 85, SEQ ID NO: 89, SEQ ID NO: 93, SEQ ID NO: 97, SEQ ID NO: 101, SEQ ID NO: 105, SEQ ID NO: 109, and SEQ ID NO: 113; preferably, the first Fab region and the second Fab region of the bispecific antibody are each independently selected from any one or more of the following sequences:

a light chain constant domain CL of SEQ ID NO: 35 and a heavy chain constant domain CH1 of SEQ ID NO: 33; or

a light chain constant domain CL of SEQ ID NO: 79 and a heavy chain constant domain CH1 of SEQ ID NO: 77; or

a light chain constant domain CL of SEQ ID NO: 55 and a heavy chain constant domain CH1 of SEQ ID NO: 53; or

a light chain constant domain CL of SEQ ID NO: 115 and a heavy chain constant domain CH1 of SEQ ID NO: 113; or

a light chain constant domain CL of SEQ ID NO: 83 and a heavy chain constant domain CH1 of SEQ ID NO: 81; or

a light chain constant domain CL of SEQ ID NO: 91 and a heavy chain constant domain CH1 of SEQ ID NO: 89; or

a light chain constant domain CL of SEQ ID NO: 107 and a heavy chain constant domain CH1 of SEQ ID NO: 105; or

a light chain constant domain CL of SEQ ID NO: 79 and a heavy chain constant domain CH1 of SEQ ID NO: 77;

preferably, the first Fab region and the second Fab region of the bispecific antibody bind to different antigens, or to two different epitopes of the same antigen;

preferably, the antigen is independently selected from any one or a combination of two of CD2, CD3, CD3E, CD4, CD11, CD11a, CD14, CD16, CD18, CD19, CD20, CD22, CD23, CD25, CD28, CD29, CD30, CD32a, CD32b, CD33, CD38, CD40, CD40L, CD52, CD54, CD56, CD64, CD80, CD147, GD3, IL-1$\alpha$, IL-1$\beta$, IL-1 R, IL-2, IL-2R, IL-4, IL-5, IL-5R, IL-6, IL-6R, IL-8, IL-9, IL-12, IL-13, IL-15, IL-17, IL-17R, IL-18, IL-23, interferon $\alpha$, interferon $\beta$, interferon $\gamma$, TNF-$\alpha$, TNF-$\beta$, TNF-R1, TNF-RII, FasL, CD27L, CD30L, 4-1BBL, TRAIL, RANKL, TWEAK, APRIL, BAFF, LIGHT, VEG1, OX40L, TRAIL receptor-1, adenosine receptor, lymphotoxin-$\beta$ receptor, TACI, BAFF-R, EPO; LFA-3, ICAM-1, ICAM-3, EpCAM, integrin $\beta1$, integrin $\beta2$, integrin $\alpha4/\beta7$, integrin $\alpha2$, integrin $\alpha3$, integrin $\alpha4$, integrin $\alpha5$, integrin $\alpha6$, integrin $\alpha v$, integrin $\alpha V\beta3$, FGFR-3, keratinocyte growth factor, VLA-1, VLA-4, L-selectin, anti-Id, E-selectin, HLA, HLA-DR, CTLA-4, T cell receptor, B7-1, B7-2, VNR integrin, TGF$\beta1$, TGF$\beta2$, eotaxin 1, Blys, complement C5, IgE, factor VII, CD64, CBL, NCA 90, EGFR, Her1, Her2/neu, Her3, Her4, tissue factor, endothelin receptor, VLA-4, hapten NP-cap, NIP-cap, E-selectin, digoxin, placental alkaline phosphatase, testicular PLAP-like alkaline phosphatase, transferrin receptor, carcinoembryonic antigen, CEACAM5, HMFG1, PEM, mucin MUC1, MUC18, heparinase I, human cardiac myosin, tumor-associated glycoprotein-72, tumor-associated antigen CA125, prostate-specific membrane antigen, high molecular weight melanoma-associated antigen, carcinoma-associated antigen, Glycoprotein IIb/IIIa, tumor-associated antigen expressing Lewis Y-associated carbohydrate, human cytomegalovirus gH envelope glycoprotein, HIVgp120, HCMV, respiratory syncytial virus RSV F, RSVF Fgp, cytokeratin tumor-associated antigen, Hep B gp120, CMV, gpIIb/IIIa, HIV IIIB gp120V3 loop, respiratory syncytial virus Fgp, herpes simplex virus gD glycoprotein, HSV gB glycoprotein, HCMV gB envelope glycoprotein, *Clostridium perfringens* toxin, CD133, CD138, OX40, GITR, PD-1, PDL1, PD-L2, CTLA-4, KIR, LAG-3, TCRα, TCRβ, TCRγ, TCRδ, VEGF, EGF, VEGFR, EGFR, EpCAM, mesothelin, glypicans, Erb1, Erb2, B7-H3, ICOS, BMP1, BMP2, BMP3B, BMP4, CSF1, GM-CSF, FGF1, FGF2, FGF3, FGF4, PDGFR, TIGIT, CS1, TWEAK, CCL1, CCL2, CCL3, CCL13, CXCL1, CXCL2, CXCL3, IP-10, fucosyl-GM1, IGF1, IGF2, IGF1R, IGF2R, RANK ligand, DLL-4, GM-CSFR, ADAMS, myostatin, PCSK9, CXCR4, MIF, and PEG2;

preferably, the first Fab region of the bispecific antibody binds to any one of CD3, CD3E, CD16, CD28, CD47, CD27, CD40, OX40, GITR, ICOS, 4-1BB, PD-1, PD-L1, GITR, TIGIT, TIM3, LAG3, B7H3, CTLA4, VISTA, BTLA, HVEM, NKG2A, and NECL;

preferably, the second Fab region of the bispecific antibody binds to any one of CD2, CD4, CD11, CD11a, CD14, CD18, CD19, CD20, CD22, CD23, CD25, CD29, CD30, CD32a, CD32b, CD33, CD38, CD52, CD54, CD56, CD64, CD80, CD147, GD3, IL-1α, IL-1β, IL-1R, IL-2, IL-2R, IL-4, IL-5, IL-5R, IL-6, IL-6R, IL-8, IL-9, IL-12, IL-13, IL-15, IL-17, IL-17R, IL-18, IL-23, interferon α, interferon β, interferon γ, TNF-α, TNF-β, TNF-R1, TNF-RII, TACI, BAFF-R, EPO; LFA-3, GPC-3, ICAM-1, ICAM-3, complement C5, IgE, factor VII, CD64, CBL, NCA 90, Her1, Her2, Her3, Her4, carcinoembryonic antigen, tumor-associated glycoprotein-72, tumor-associated antigen CA125, prostate-specific membrane antigen, high molecular weight melanoma-associated antigen, carcinoma-associated antigen, and tumor-associated antigen expressing Lewis Y-associated carbohydrate;

preferably, an antigen to which the first Fab region of the bispecific antibody binds is CD3, and an antigen to which the second Fab region binds is GPC3;

preferably, the bispecific antibody has a first heavy chain and a first light chain binding to CD3, and a second heavy chain and a second light chain binding to GPC3; and

preferably, the bispecific antibody is selected from any one of following amino acid sequences:

the first heavy chain has an amino acid sequence set forth in SEQ ID NO: 129, and the first light chain has an amino acid sequence set forth in SEQ ID NO: 127; the second heavy chain has an amino acid sequence set forth in SEQ ID NO: 133, and the second light chain has an amino acid sequence set forth in SEQ ID NO: 131; or

the first heavy chain has an amino acid sequence set forth in SEQ ID NO: 145, and the first light chain has an amino acid sequence set forth in SEQ ID NO: 143; the second heavy chain has an amino acid sequence set forth in SEQ ID NO: 149, and the second light chain has an amino acid sequence set forth in SEQ ID NO: 147; or

the first heavy chain has an amino acid sequence set forth in SEQ ID NO: 137, and the first light chain has an amino acid sequence set forth in SEQ ID NO: 135; the second heavy chain has an amino acid sequence set forth in SEQ ID NO: 141, and the second light chain has an amino acid sequence set forth in SEQ ID NO: 139; or

the first heavy chain has an amino acid sequence set forth in SEQ ID NO: 153, and the first light chain has an amino acid sequence set forth in SEQ ID NO: 151; the second heavy chain has an amino acid sequence set forth in SEQ ID NO: 157, and the second light chain has an amino acid sequence set forth in SEQ ID NO: 155.

5. The bispecific antibody according to claim 4, wherein the bispecific antibody is derived from IgG, IgA, IgM, IgE, or IgD; preferably, the bispecific antibody is derived from IgG1, IgG2, IgG3, or IgG4.

6. A nucleic acid molecule, **characterized in that** the nucleic acid molecule encodes the antibody or antibody fragment according to any one of claims 1 to 3 and/or the bispecific antibody according to any one of claims 4 to 5.

7. An expression vector, **characterized in that** the expression vector comprises the nucleic acid molecule according to claim 6.

8. A recombinant cell, **characterized in that** the recombinant cell expresses the antibody or antibody fragment according to any one of claims 1 to 3 and/or the bispecific antibody according to any one of claims 4 to 5,

wherein preferably, the nucleic acid molecule according to claim 6 is integrated into a genome of the recombinant cell;

preferably, the recombinant cell comprises the expression vector according to claim 7.

9. A method for preparing the antibody or antibody fragment according to any one of claims 1 to 3, **characterized by** comprising steps of:

linking a nucleic acid molecule encoding the antibody or antibody fragment into a plasmid, transferring a resultant into competent cells, and subsequently selecting and screening monoclonal cells;

extracting an expression vector from screened positive clone, transferring a resultant into recipient cells, and obtaining recombinant cells by screening; and

culturing the recombinant cells, collecting a culture supernatant, and obtaining the antibody or antibody fragment by isolation and purification.

10. An antibody conjugate, **characterized in that** the antibody conjugate comprises the antibody or antibody fragment according to any one of claims 1 to 3 and/or the bispecific antibody according to any one of claims 4 to 5, and a conjugated marker,

wherein preferably, the conjugated marker comprises any one or a combination of at least two of a cytotoxin, a radioisotope, a fluorescent marker, a luminescent substance, a chromogenic substance, or an enzyme.

11. A pharmaceutic composition, **characterized in that** the pharmaceutical composition comprises any one or a combination of at least two of the antibody or antibody fragment according to any one of claims 1 to 3, the bispecific antibody according to any one of claims 4 to 5, and the antibody conjugate according to claim 10,

wherein preferably, the pharmaceutical composition further comprises any one or a combination of at least two of a pharmaceutically acceptable carrier, a surfactant, a diluent, and an excipient.

12. Use of any one or a combination of at least two of the antibody or antibody fragment according to any one of claims 1 to 3, the bispecific antibody according to any one of claims 4 to 5, the antibody conjugate according to claim 10, or the pharmaceutical composition according to claim 11 in a preparation of a medicament for the treatment of a disease,

wherein preferably, the disease includes cancer.

13. Use of any one or a combination of at least two of the antibody or antibody fragment according to any one of claims 1 to 3, the bispecific antibody according to any one of claims 4 to 5, the antibody conjugate according to claim 10, or the pharmaceutic composition according to claim 11 in treatment of a disease,

wherein preferably, the disease includes cancer.

14. A method for treating a disease, **characterized in that** the method comprises: administering any one or a combination of at least two of the antibody or antibody fragment according to any one of claims 1 to 3, the bispecific antibody according to any one of claims 4 to 5, the antibody conjugate according to claim 10, or the pharmaceutic composition according to claim 11 to a subject in need thereof,

wherein preferably, the disease includes cancer.

**FIG. 1**

FIG. 2

FIG. 3A

**FIG. 3B**

**FIG. 3C**

**FIG. 3D**

FIG. 3E

FIG. 3F

## GPC3 ELISA

FIG. 4A

## CD3 ELISA

FIG. 4B

## GPC3 ELISA

FIG. 4C

## CD3 ELISA

FIG. 4D

**GPC3 ELISA**

FIG. 5A

**CD3 ELISA**

FIG. 5B

**GPC3 ELISA**

FIG. 5C

CD3 ELISA

FIG. 5D

**FIG. 6**

FIG. 7A

FIG. 7B

FIG. 8A

EP 4 455 165 A1

**Jurkat/NFAT-Luc+HepG2 (10:1)**

FIG. 8B

**PBMC （#137） +HepG2 （E:T=20:1）**

FIG. 9A

**PBMC （#172） +HepG2 （E:T=20:1）**

FIG. 9B

PBMC (#171) +HepG2 (E:T=20:1)

FIG. 9C

## INTERNATIONAL SEARCH REPORT

| International application No. |
| --- |
| **PCT/CN2022/120996** |

**A. CLASSIFICATION OF SUBJECT MATTER**

C07K 16/46(2006.01)i;  C12N 15/13(2006.01)i;  A61K 39/395(2006.01)i;  C07K 16/00(2006.01)i;  A61P 27/16(2006.01)i

According to International Patent Classification (IPC) or to both national classification and IPC

**B. FIELDS SEARCHED**

Minimum documentation searched (classification system followed by classification symbols)

C07K C12N A61K A61P

Documentation searched other than minimum documentation to the extent that such documents are included in the fields searched

Electronic data base consulted during the international search (name of data base and, where practicable, search terms used)

CNTXT; WPABSC; WPABS; ENTXT; ENTXTC; VEN; CJFD; ISI web of Science; Genbank; EBI; ENA; 万方, Wanfang; CNKI; STN, 中国专利生物序列检索, China Patent Biological Sequence Search System: 抗体, 双特异性抗体, 重/轻链, 轻/重链, 重链, 轻链, 优先配对, 正确配对, 配对, 突变, 修饰, 界面, CH1, CL, 疏水, 电荷, v190, 错配, s176, s186, s188, t178, s174, 186, antibod+, 178, 188, 176, 190, 186, 174, bispecific antibody, bsab, heavy chain, light chain, match, pair, mutant, interaction, 序列检索, sequence search

**C. DOCUMENTS CONSIDERED TO BE RELEVANT**

| Category* | Citation of document, with indication, where appropriate, of the relevant passages | Relevant to claim No. |
| --- | --- | --- |
| PX | CN 114106192 A (GUANGZHOU EISHI BIOMEDICAL TECHNOLOGY CO., LTD.) 01 March 2022 (2022-03-01) claims 1-12 | 1-14 |
| X | CN 106661121 A (ZYMEWORKS INC.) 10 May 2017 (2017-05-10) claims 1-3, 6-7, 11-13, 33, 56-57, description, paragraphs 147, 250, 133-136 | 1-14 |
| X | TW 201602135 A (PFIZER) 16 January 2016 (2016-01-16) description, page 36, paragraphs 3-4 from the bottom, page 37, paragraphs 3-4, page 42, last paragraph to page 43, paragraph 1, pages 83-93 | 1-14 |
| A | CN 103797033 A (CENTRE NATIONAL DE LA RECHERCHE SCIENTIFIQUE et al.) 14 May 2014 (2014-05-14) description, paragraphs 18-24 | 1-14 |
| A | CN 111094355 A (BIOMUNEX PHARMACEUTICALS) 01 May 2020 (2020-05-01) description, paragraphs 34, 56 | 1-14 |

☑ Further documents are listed in the continuation of Box C.    ☑ See patent family annex.

| | |
| --- | --- |
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **08 December 2022** | **19 December 2022** |

| Name and mailing address of the ISA/CN | Authorized officer |
| --- | --- |
| **China National Intellectual Property Administration (ISA/ CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2022/120996**

### C.    DOCUMENTS CONSIDERED TO BE RELEVANT

| Category* | Citation of document, with indication, where appropriate, of the relevant passages | Relevant to claim No. |
|---|---|---|
| A | BONISCH, M. et al. "Novel CH1:CL Interfaces that Enhance Correct Light Chain Pairing in Heterodimeric Bispecific Antibodies" <br> *Protein Engineering, Design & Selection,* Vol. 30, No. (9), 31 August 2017 (2017-08-31), 685–696 | 1-14 |
| A | GOLAY, J. et al. "Design and Validation of a Novel Generic Platform for the Production of Tetravalent IgG1-like Bispecific antibodies" <br> *The Journal of Immunology,* Vol. 196, No. (7), 01 April 2016 (2016-04-01), 3199–3211 | 1-14 |

**INTERNATIONAL SEARCH REPORT**

| International application No. |
| --- |
| **PCT/CN2022/120996** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a.  ☑  forming part of the international application as filed:

          ☑  in the form of an Annex C/ST.25 text file.

          ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

          ☐  in the form of an Annex C/ST.25 text file (Rule 13*ter*.1(a)).

          ☐  on paper or in the form of an image file (Rule 13*ter*.1(b) and Administrative Instructions, Section 713).

2. ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    [1]  The actually submitted sequence listing is an XML file in Standard ST.26.

**INTERNATIONAL SEARCH REPORT**

| International application No. |
| --- |
| **PCT/CN2022/120996** |

| **Box No. II** | **Observations where certain claims were found unsearchable (Continuation of item 2 of first sheet)** |
| --- | --- |

This international search report has not been established in respect of certain claims under Article 17(2)(a) for the following reasons:

1. ☑ Claims Nos.: **14**
because they relate to subject matter not required to be searched by this Authority, namely:

> [1] Claim 14 relates to a method for treating cancers, which belongs to a method for the treatment of a living human or animal body, and belongs to the subject matter requiring no search by the International Searching Authority as defined in PCT Rule 39.1(iv). The present international search is carried out on the basis of the use of a bispecific antibody and an antibody fragment and an antibody conjugate in the preparation of a drug for treating cancers.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

## INTERNATIONAL SEARCH REPORT
### Information on patent family members

International application No.

**PCT/CN2022/120996**

| Patent document cited in search report | | | Publication date (day/month/year) | Patent family member(s) | | | Publication date (day/month/year) |
|---|---|---|---|---|---|---|---|
| CN | 114106192 | A | 10 May 2017 | JP | 2018502550 | A | 01 February 2018 |
| | | | | JP | 2020202848 | A | 24 December 2020 |
| CN | 106661121 | A | 10 May 2017 | JP | 2018502550 | A | 01 February 2018 |
| | | | | JP | 2020202848 | A | 24 December 2020 |
| TW | 201602135 | A | 16 January 2016 | EP | 3143043 | A2 | 22 March 2017 |
| | | | | CA | 2891714 | A1 | 16 November 2015 |
| | | | | JP | 2021106598 | A | 29 July 2021 |
| | | | | US | 2020123260 | A1 | 23 April 2020 |
| | | | | US | 2018179285 | A1 | 28 June 2018 |
| | | | | JP | 2017521361 | A | 03 August 2017 |
| | | | | WO | 2015173756 | A2 | 19 November 2015 |
| CN | 103797033 | A | 14 May 2014 | HR | P20201191 | T1 | 13 November 2020 |
| | | | | WO | 2013005194 | A2 | 10 January 2013 |
| | | | | KR | 20140074274 | A | 17 June 2014 |
| | | | | US | 2020385490 | A1 | 10 December 2020 |
| | | | | EP | 2543680 | A1 | 09 January 2013 |
| | | | | LT | 2729499 | T | 25 August 2020 |
| | | | | PL | 2729499 | T3 | 08 March 2021 |
| | | | | US | 2018022829 | A1 | 25 January 2018 |
| | | | | HU | E051620 | T2 | 29 March 2021 |
| | | | | JP | 2014522644 | A | 08 September 2014 |
| | | | | US | 2021040235 | A1 | 11 February 2021 |
| | | | | US | 2014242076 | A1 | 28 August 2014 |
| | | | | ES | 2816701 | T3 | 05 April 2021 |
| | | | | EP | 2729499 | A2 | 14 May 2014 |
| | | | | PT | 2729499 | T | 03 August 2020 |
| | | | | MX | 2014000234 | A | 04 September 2014 |
| | | | | EP | 3872095 | A1 | 01 September 2021 |
| | | | | SI | 2729499 | T1 | 31 December 2020 |
| | | | | DK | 2729499 | T3 | 03 August 2020 |
| | | | | CA | 3099509 | A1 | 10 January 2013 |
| | | | | CA | 2841039 | A1 | 10 January 2013 |
| CN | 111094355 | A | 01 May 2020 | JP | 2020515253 | A | 28 May 2020 |
| | | | | EP | 3601366 | A1 | 05 February 2020 |
| | | | | RU | 2019134211 | A | 28 April 2021 |
| | | | | IL | 269559 | A | 28 November 2019 |
| | | | | AU | 2018241881 | A1 | 07 November 2019 |
| | | | | CA | 3057567 | A1 | 04 October 2018 |
| | | | | KR | 20190141154 | A | 23 December 2019 |
| | | | | BR | 112019019998 | A2 | 28 April 2020 |
| | | | | SG | 11201908825T | A | 30 October 2019 |
| | | | | WO | 2018178101 | A1 | 04 October 2018 |
| | | | | ZA | 201906981 | B | 30 September 2020 |
| | | | | MX | 2019011585 | A | 17 February 2020 |
| | | | | US | 2020299413 | A1 | 24 September 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111585813
- CN 102471378 B

**Non-patent literature cited in the description**

- **SPIESS et al.** *Molecular Immunology,* 2015, vol. 67 (2), 95-106
- **RIDGWAY et al.** *Protein Engineering,* 1996, vol. 9 (7), 617-21
- **MERCHANT et al.** *Nature Biotechnology,* 1998, vol. 16 (7), 677-681
- **GUNASEKARAN et al.** *Journal of Biological Chemistry,* 2010, vol. 285 (25), 19637-19646
- **KREUDENSTEIN et al.** *, mAbs,* 2013, vol. 5 (5), 646-654
- **LEAVER-FAY et al.** *, Structure,* 2016, vol. 24 (4), 641-651
- **KABAT, E. A. et al.** Sequences of Proteins of Immunological Interest. NIH, 1991
- **SAMBROOK JF, E. F. et al.** Molecular cloning: a laboratory manual. Cold Spring Harbor Laboratory Press, 2012
- **HUST et al.** II Construction of Synthetic Antibody Phage-Display Libraries[J. *Methods in Molecular Biology-Phage Display,* 2018, vol. 1701